Europäisches Patentamt

European Patent Office

Office européen des brevets

(19)

(11) Publication number: 0 205 038

A1

(12) **EUROPEAN PATENT APPLICATION**

(21) Application number: 86107251.0

(22) Date of filing: 28.05.86

(51) Int. Cl.⁴: **C 12 P 21/02**
C 12 N 15/00, A 61 K 37/02
C 12 N 1/20
//(C12N1/20, C12R1:19)

(30) Priority: 29.05.85 JP 115564/85
29.05.85 JP 115565/85

(43) Date of publication of application:
17.12.86 Bulletin 86/51

(84) Designated Contracting States:
AT BE CH DE FR GB IT LI LU NL SE

(71) Applicant: Suntory Limited
1-40 Dojimahama 2-chome Kita-ku
Osaka-shi Osaka-fu 530(JP)

(72) Inventor: Nishihara, Tatsuro
1-B-902, Satsukigaoka-Nishi
Suita-shi Osaka(JP)

(72) Inventor: Tsujimoto, Masafumi
2-2-4-601, Minase Shimamoto-cho
Mishima-gun Osaka(JP)

(72) Inventor: Tsuruoka, Nobuo
18-7, Inaba-cho
Ibaraki-shi Osaka(JP)

(72) Inventor: Kubota, Ichiro
2-1-603, Mizuo
Ibaraki-shi Osaka(JP)

(72) Inventor: Oshima, Takehiro
17-10-383, Besshohon-machi
Takatsuki-shi Osaka(JP)

(72) Inventor: Tanaka, Shoji
1-8-46-603, Minamikaneden
Suita-shi Osaka(JP)

(72) Inventor: Matsukura, Shigekazu
10-29-305, Ogawa-cho
Ibaraki-shi Osaka(JP)

(74) Representative: Lehn, Werner, Dipl.-Ing. et al,
Hoffmann, Eitle & Partner Patentanwälte Arabellastrasse
4 (Sternhaus)
D-8000 München 81(DE)

(54) Polypeptide, process for preparing it, microorganism and pharmaceutical use.

(57) A novel polypeptide having antitumor activity wherein the first 23 NH₂-terminal amino acid sequence of its monomeric form is

Ser - Arg - Thr - Pro - Ser - Asp - Lys - Pro - Val - Ala - His - Val - Val - Ala - Asn - Pro - Gln - Ala - Glu - Gly - Gln - Leu - Gln

and which has the following characteristics: (a) a molecular weight of $17,000 \pm 1,500$ as calculated from data obtained by SDS polyacrylamide electrophoresis; (b) an isoelectric point of $5.2 \pm 0.2$ as determined by chromatofocusing; (c) a cytocidal activity against L-929 cells of $1 \times 10^7 - 10 \times 10^7$ units/mg protein as defined hereinafter; and (d) ability to adsorb on a dye adsorption affinity carrier and preparation or purification thereof are disclosed. This novel type of polypeptide has excellent antitumor activities, and is obtained by use of the recombinant DNA technique, or by special purification from the culture of activated human machrophage or human macrophage-like cells.

EP 0 205 038 A1

## POLYPEPTIDE, PROCESS FOR PREPARING IT, MICROORGANISM AND PHARMACEUTICAL USE

The invention relates to a novel polypeptide having antitumor activities, and a process for preparing said polypeptide. Specifically, the invention relates to a process for preparing a novel polypeptide having antitumor or tumor necrosis activities by use of the recombinant DNA technique or by purification from the culture of activated human macrophage or human macrophage-like cells, and also to a novel polypeptide obtained by the process and antitumor or tumor necrosis preparations containing said polypeptide.

Increasing knowledge has been accumulated about the production of trace amounts of biologically important substances, such as interferons (IFN), interleukine 2 (IL2), lymphotoxin (LT) and tumor necrosis factor (TNF), from such various components in body fluids, particularly blood, as leukocytes, lymphocytes and macrophages. As a result, many researchers have been making attempts at producing these useful substances either by cultivating cell lines or fused cells capable of producing such substances or by culturing microorganisms or animal cells constructed by the recombinant DNA technology.

The existence of a substance having tumor killing activity has been reported in association with the results of _in vitro_ and _in vivo_ tests. Carswell et al. first reported in Proc. Natl. Acad. Sci., U.S.A. _72_, 3666 - 3670 (1975) the discovery of this substance in the serum of endotoxin-injected mice, rats and rabbits, previously sensitized with Mycobacterium bovis strain Bacillus Calmelte-Guérin (BCG) or other immunostimulatory agents. This activity of the serum was named tumor necrosis factor (hereunder referred to as TNF). Today, the production of TNF is considered to derive from activated macrophages in the organism (Ruff, M. R. & Gifford, G. E. in Lymphokine reports (Pick, E., ed.) Vol. 2, pp. 235 - 272, Academic Press, New York, 1981), and as a matter of fact, the TNF activity can be obtained in the culture supernatant of BCG- or endotoxin-treated monocytic cells.

TNF causes hemorrhagic necrosis of various tumors with no apparent effect on the host and displays cytocidal and cytostatic activities against several transformed cells (those which have differentiated to tumors) without affecting normal cells. Because of these features, TNF holds great promise as antitumor agents.

Various attempts have been made to obtain human derived substances having TNF activity, and they include efforts to obtain native human TNF by cultivation of human macrophage-like cells and those to obtain synthetic human TNF by culturing microorganisms created by the recombinant DNA technology. As for the recovery of natural human TNF, Unexamined Published Japanese Patent Application No. 30688/1985 discloses a method depending on the treatment of human macrophage-like cell lines, HL-60, THP-1 or Mono-1-207 with a variety of inducers. Pennica, D. et al. (Nature, 312, 724 - 729, 1984) and Aggarwal, B. B. et al. (J. Biol. Chem., 260, 2345 - 2354, 1985) of Genentech, Inc., California, U.S.A. reported a method of inducing and purifying human TNF from HL-60 cells. Wang, A. M. et al. (Science, 228, 149 - 154, 1985) of Cetus Corp., California, U.S.A. reported another method for inducing and purifying human TNF from HL-60 cells. The method of purifying human TNF and its physicochemical properties are best described by the above-mentioned scientists of Genentch, Inc.

According to their reports, native human TNF could be obtained from culture supernatants of the HL-60 promyelocytic leukemia cell induced by 4β-phorbal 12β-myristate 13α-acetate (PMA) after subjecting them to a purification scheme consisting of controlled-pore glass beads, DEAE-cellulose 53 chromatography, Mono Q-fast protein liquid chromatography and preparative reverse-phase high performance liquid chromatography. The purified human TNF had a molecular weight (MW) of 17,000 as estimated by SDS polyacrylamide gel electrophoresis (hereunder SDS PAGE) and MW of 45,000 $\pm$ 6,000 as deduced from high pressure gel permeation chromatography using a TSK G2000 SW column. Based on these data, the authors suggested that human TNF would occur naturally as a dimeric or trimeric polypeptide, and that the molecular weights of 34,000 - 140,000 previously reported by Matthews, N. (Immunology, 44, 135 - 142, 1981) and Nissen-Meyers, J. & Hammerstrom, J. (Infect. Immun., 38, 67 - 73, 1982) may have resulted from differences caused by oligomerization under natural conditions. By chromatofocusing on a Mono P column, the purified human TNF was found to have an isoelectric point (pI) of 5.2 - 5.4.

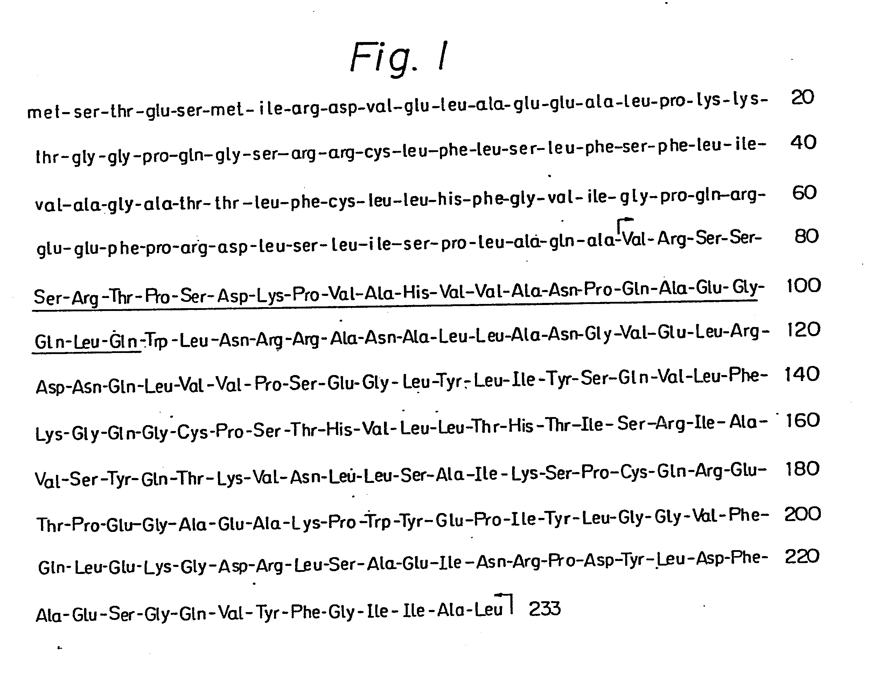

Furthermore, as a result of amino acid sequence analysis, the purified human TNF was found to be a polypeptide having an N terminus beginning with Val - Arg - Ser - Ser. According to the analysis of cDNA clone made from mRNA that was prepared from PMA-induced HL-60 cells, the natural mature human TNF monomer was assumed to be a polypeptide having a calculated molecular weight of 17,356 that was composed of 157 amino acids obtained by processing (deleting) the first N-terminal 76 amino acid sequence from a precursor polypeptide consisting of 233 amino acids (see Fig. 1). In view of the results of SDS PAGE and N-terminal amino acid sequence of the purified human TNF from HL-60 cells, the natural human TNF does not occur in a heterogeneous state as lymphotoxin or γ-interferon (both of which occur naturally as polypeptides of various sizes as a consequence of N-terminal and C-terminal proteolysis, respectively) but occurs as a homogeneous monomer. In other words, mature human TNF monomer is a polypeptide that is formed as part of its precursor (see Fig. 1) ... Ala-Gln-Ala-Val-Arg-Ser-Ser-Ser-Arg-Thr- ... and which has been cleaved at the position indicated by the vertical arrow; no significant post-translational processing such as proteolysis or glycosidation will occur in the HL-60 cell line.

Another prior art reference that describes the amino acid sequence of human TNF is Shirai, T., et al., Nature, 313, 803 - 806, 1985 which reports the production of human TNF-like polypeptide with 155 amino acids by the recombinant DNA technology. Shirai et al. analyzed the DNA sequence of cDNA clone of human TNF but they did not predict the whole amino acid sequence of mature human TNF by determining the N-terminal amino acid sequence of naturally occurring human TNF polypeptide as was done by the scientist group of Genentech, Inc. Instead, they used as a reference the N-terminal amino acid sequence of rabbit TNF disclosed in Unexamined Published Japanese Patent Application No. 19719/1985, and simply estimated that human TNF would be a polypeptide with a calculated molecular weight of 17,099 that consisted of 155 amino acids with a sequence beginning with Ser-Ser-Ser-Arg-Thr-Pro. Shirai et al. presumed that the purified human TNF they produced from $E.$ $coli$ would have almost the same or an identical structure to the natural human TNF. Shirai et al. obtained the purified polypeptide from the supernatant cell lysate of a culture of recombinant DNA $E.$ $coli$ by a purification scheme consisting of chromatography on DEAE-Sepharose CL-6B column, heating at $60°C$ for 30 minutes, chromatography on another DEAE-Sepharose CL-6B column, and column chromatography on Sephacryl S-200. The polypeptide had molecular weights of 45,000 as determined from chromatography on Sephacryl S-200 column, and 17,000 as determined by SDS PAGE. Isoelectric focusing showed that the polypeptide's isoelectric point was 6.0.

The inventors succeeded in purifying a novel polypeptide having TNF activities different from the above polypeptide from a culture solution of activated macrophage-like cell U-937 (ATCC deposit No.: CRL1593). The polypeptide is different from the polypeptide already reported in the above literatures in terms of not only amino acid sequence but also physical and chemical properties (although the amino acid sequence at the carboxyl terminus is the same). The new polypeptide was expected to be a useful antitumor agent different from conventional agents.

Furthermore, the inventors made earnest study so as to prepare a large amount of said novel polypeptide (refer to $\Delta N4$TNF hereunder) in good efficiencies and found that an E. coli transformed by a plasmid constructed as follows is cultivated to produce $^{\Delta N4}$TNF. Said plasmid is constructed by joining a promoter derived from an E. coli gene, or a λ phage or a T4 phage promoter region, and the ribosome binding (or SD) sequence with a DNA sequence coding for $^{\Delta N4}$TNF at a site upstream of the DNA sequence, also joining a transcription terminator coding for the termination of transcription just downstream of said $^{\Delta N4}$TNF gene interrupted by a translation termination codon, and inserting the joined DNA sequence into a plasmid derived form pBR322, from which the rop (repressor of primer) region coding for the replication of the plasmid may be deleted, if desired.

Fig. 1 shows the amino acid sequence of human TNF precursor deduced from the nucleotide sequence of human TNF cDNA clones prepared by Pennica, D. et al. (Nature, 312, 724 - 729, 1984) wherein the amino acid sequence of mature TNF (composed of 157 amino acids) deduced by Pennica, E. et al. is bracketed by ⌐→ and ←⌐, and the underlined portion represents the $NH_2$-terminal amino acid sequence determined by the amino acid sequencing of the novel polypeptide purified in the present invention;

Figs. 2 to 6 show chromatograms of U-937 produced-TNF obtained in the respective steps of purification used in the present invention, wherein:

Fig. 2 shows the results of column chromatography on DEAD-Sepharose;

Fig. 3 shows the results of chromatofocusing;

Fig. 4 shows the results of column chromatography on Phenyl-Sepharose;

Fig. 5 shows the results of column chromatography on dye adsorption affinity carrier, Matrex Blue A; and

Fig. 6 shows the results of column chromatography on gel permeation carrier;

Fig. 7 shows SDS PAGE patterns for the polypeptide, U-937 TNF, obtained in the present invention and the polypeptide, Recombinant TNF, composed of 157 amino acids as shown in Fig. 1 which was purified from a culture of recombinant E. coli cells;

Fig. 8 is a scheme illustrating a process for constructing plasmid pPLT4$^{\Delta N4}$TNF from plasmid pBR322-PL-T4-hTNF;

Figs. 9-a and 9-b are scheme illustrating a process for constructing plasmid pPLT4$^{\Delta N4}$TNFST8 for use in producing $^{\Delta N4}$TNF from plasmid pPLT4$^{\Delta N4}$TNF obtained in Fig. 8;

Fig. 10 is a scheme illustrating a process for constructing pT4$^{\Delta N4}$TNFST8$^{rop-}$, another plasmid for producing $^{\Delta N4}$TNF;

Fig. 11 is a diagram illustrating a process for constructing pIN5$^{\Delta N4}$TNFST8$^{rop-}$ which is another plasmid for producing $^{\Delta N4}$TNF;

Figs. 12 to 14 are graphs illustrating the results of various types of chromatography at each purification process for $^{\Delta N4}$TNF, wherein;

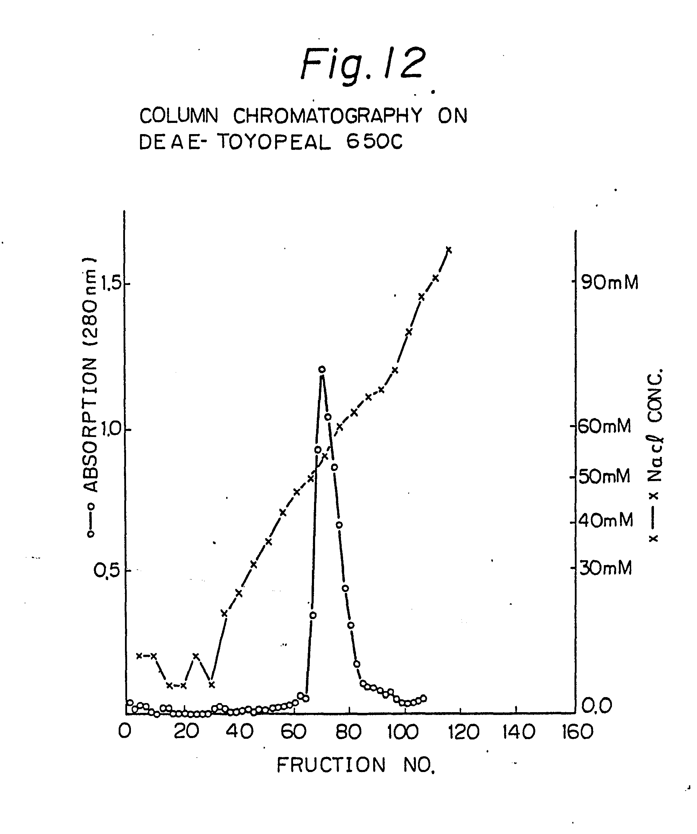

Fig. 12 shows a DEAE-Toyopearl 650C column chromatogram;

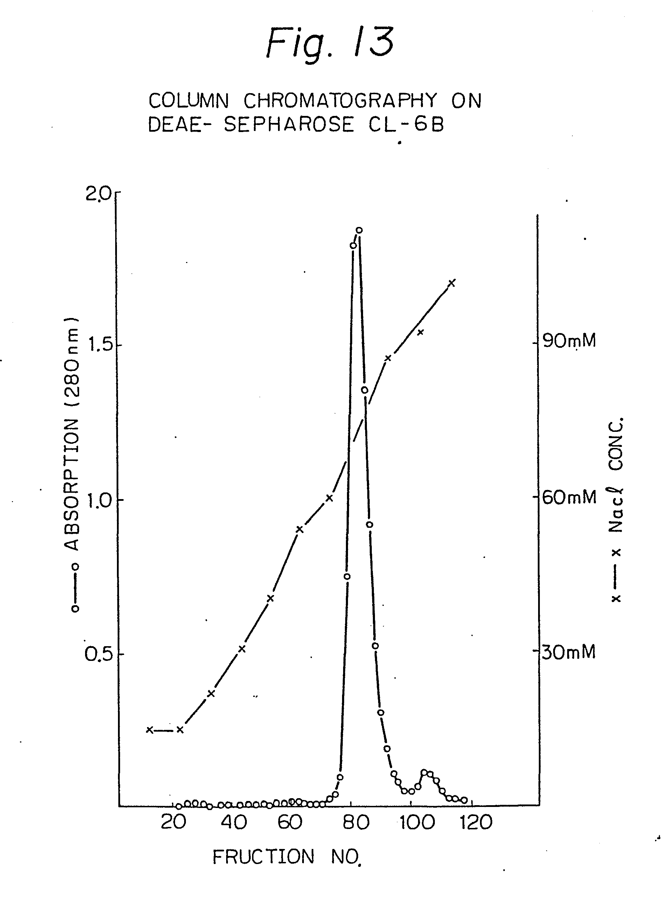

Fig. 13 shows DEAE-Sepharose CL-6B column chromatogram; and

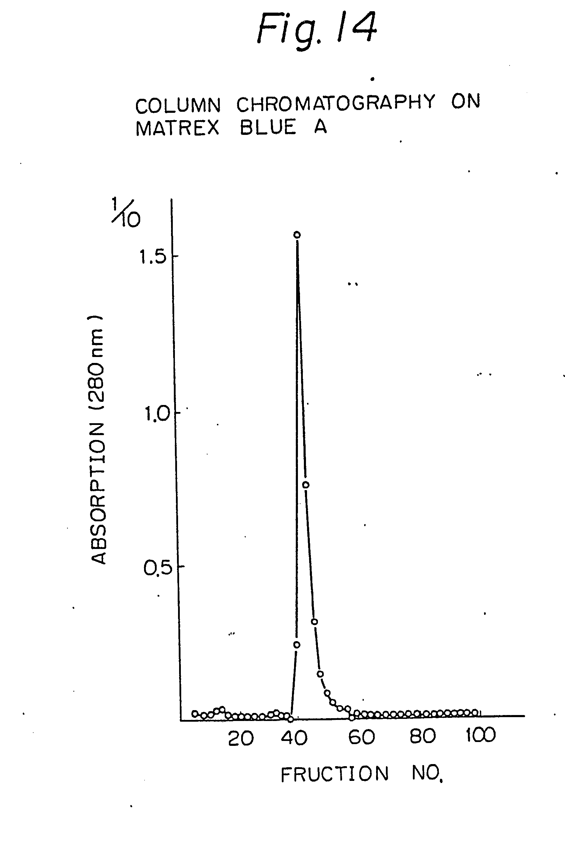

Fig. 14 shows chromatogram on Matrex Blue A, a dye adsorption affinity carrier;

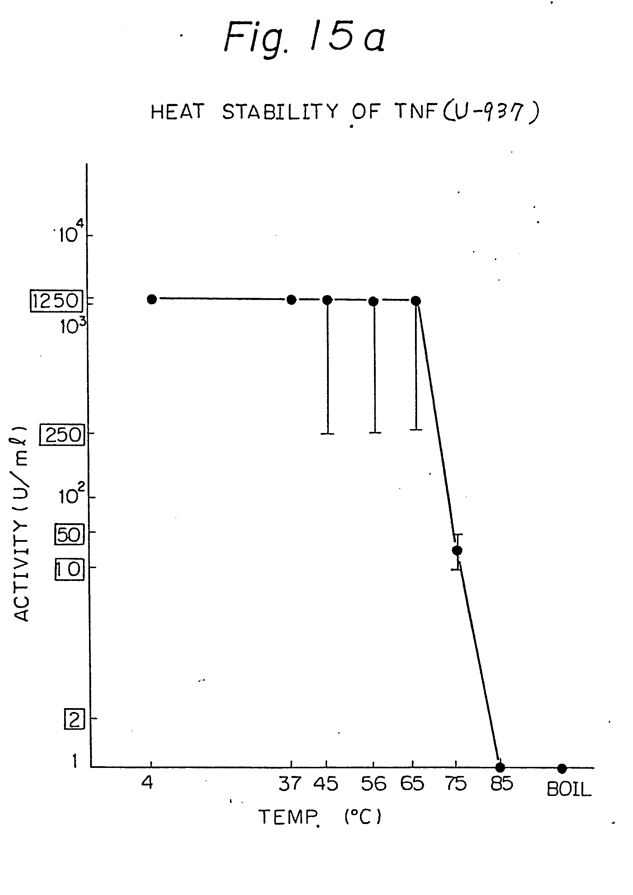

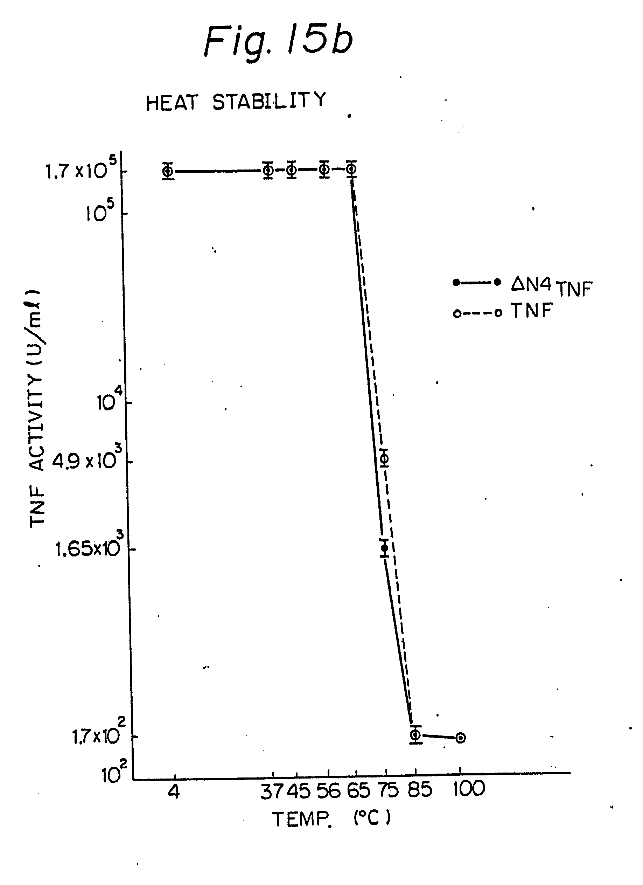

Figs. 15-a and 15-b are graphs showing the heat stabilities of TNF activity in the hollow fiber concentrate of a culture broth of U-937 cells that was obtained in Example 1-3-a) and of ΔTNF activity, respectively;

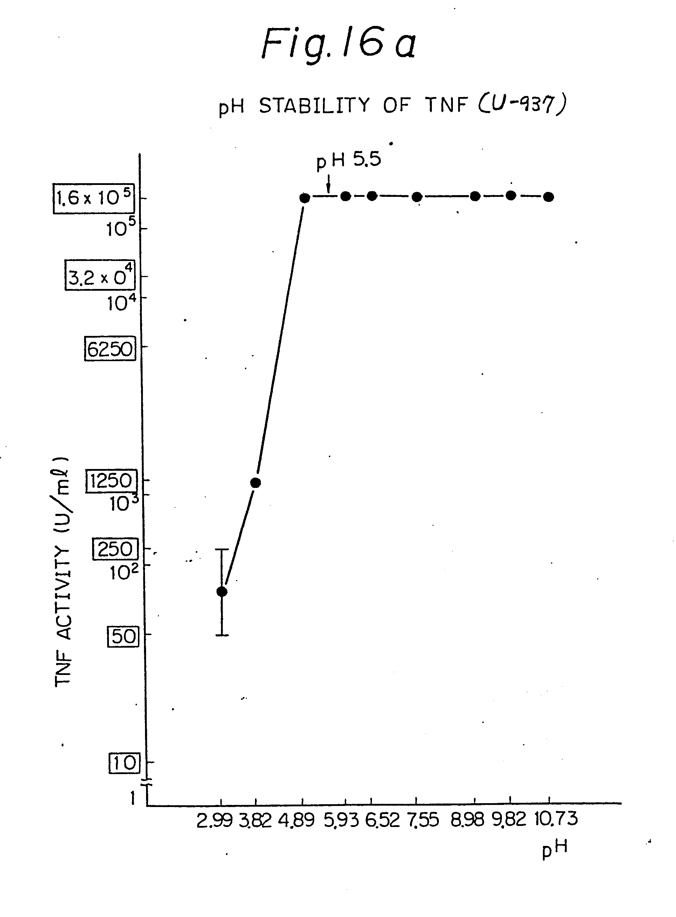

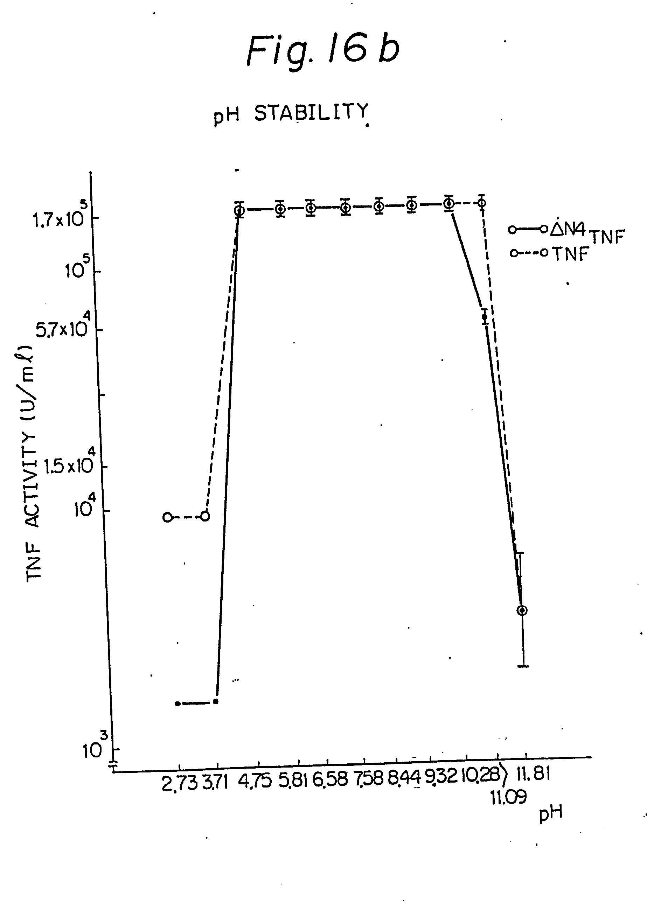

Figs. 16-a and 16-b are graphs showing the pH stabilities of TNF activity in the concentrate used in obtaining the data shown in Fig. 15-a, and of ΔTNF activity in Fig. 15-b, respectively;

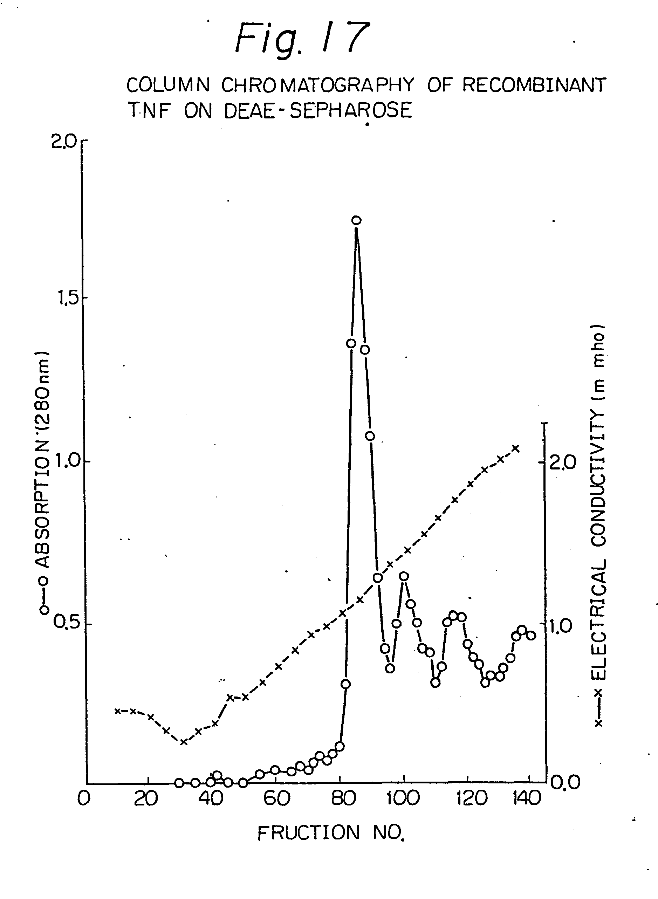

Figs. 17 to 20 show chromatograms obtained in the respective steps of purification of TNF from a culture of recombinant E. coli cells having the ability to produce a polypeptide made of 157 amino acids as bracketed by $\lceil$ and $\rceil$ in Fig. 1, wherein:

Fig. 17 shows the results of column chromatography on DEAE-Sepharose;

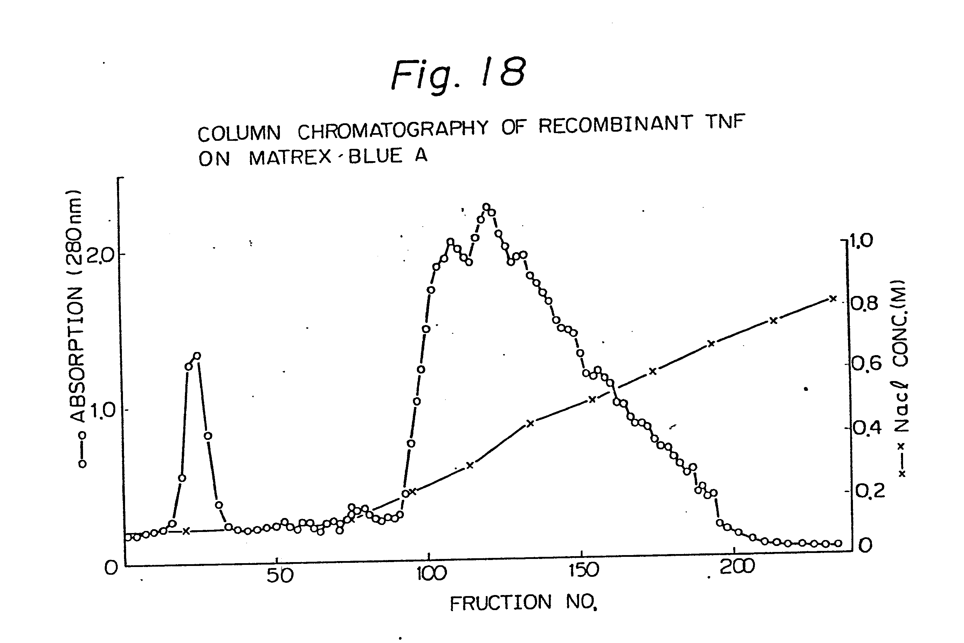

Fig. 18 shows the results of column chromatography on dye adsorption affinity carrier, Matrex Blue A;

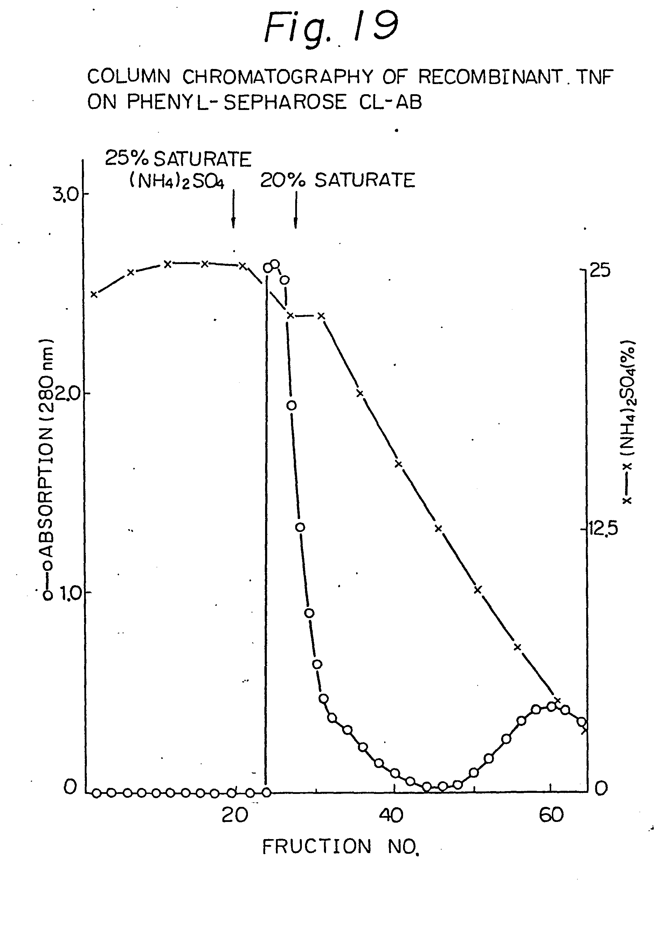

Fig. 19 shows the results of column chromatography on Phenyl-Sepharose; and

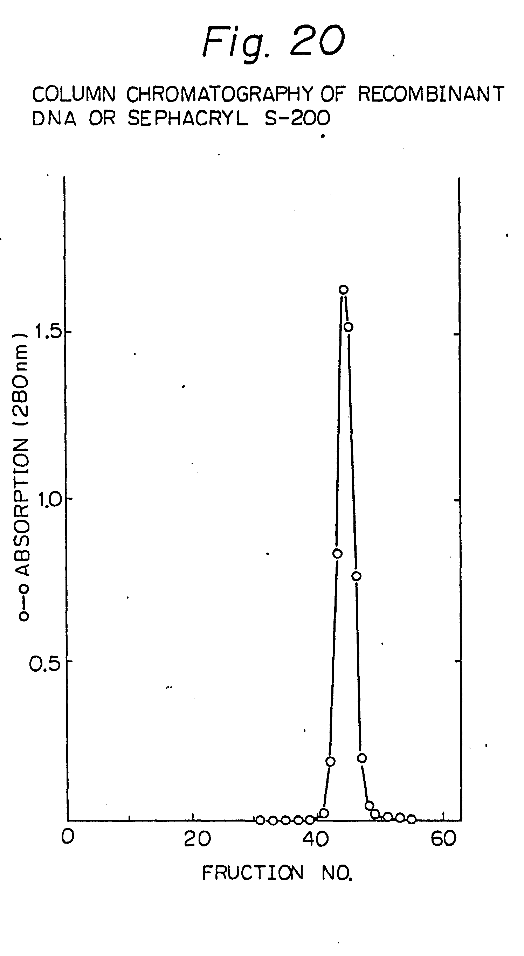

Fig. 20 shows the results of column chromatography on gel permeation carrier;

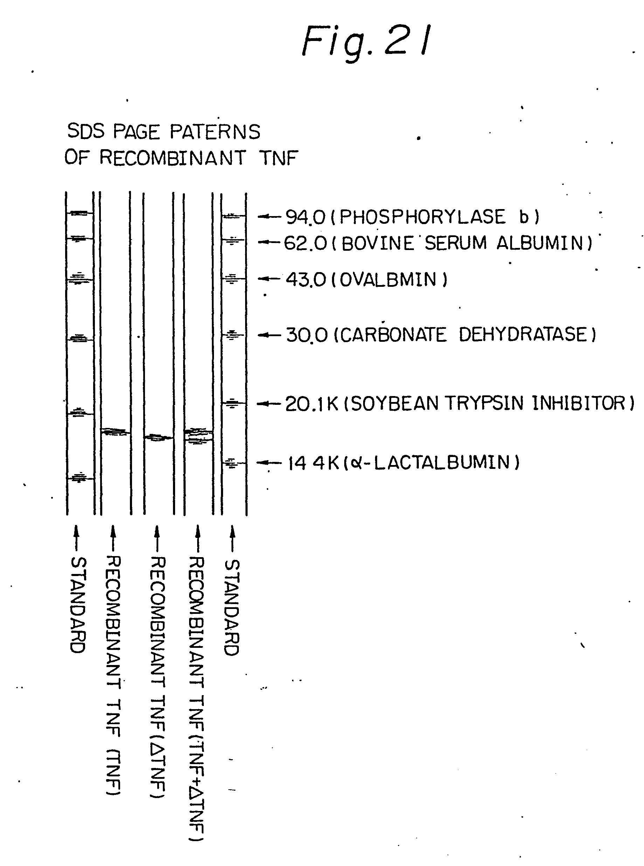

Fig. 21 shows SDS PAGE patterns for the polypeptide, Recombinant TNF, composed of 157 amino acids and purified from a culture of recombinant E. coli cells (indicated by ⌐→ and ←⌐ in Fig. 1) and the polypeptide of the present invention, Recombinant ΔTNF, also purified from a culture of recombinant E. coli cells; and

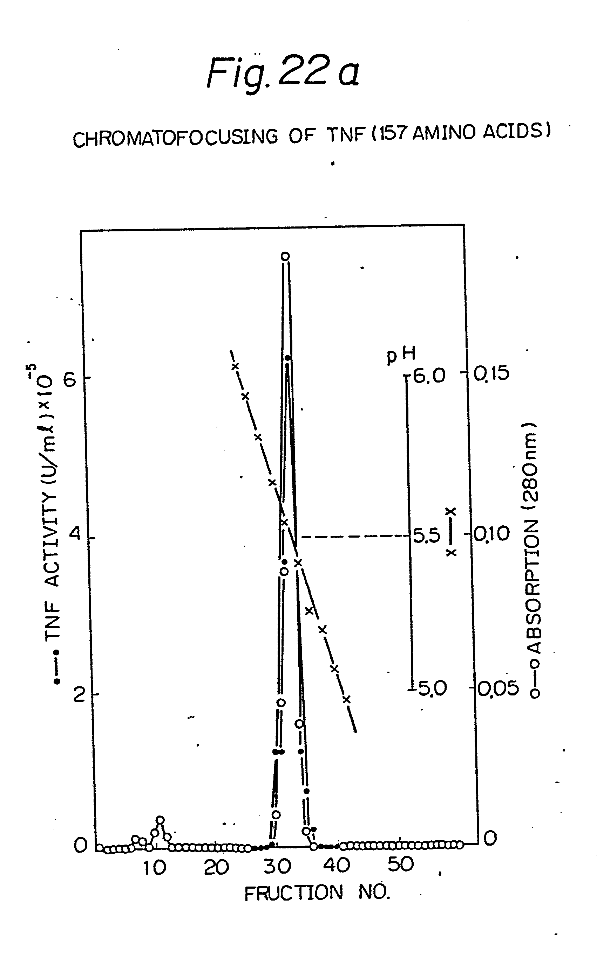

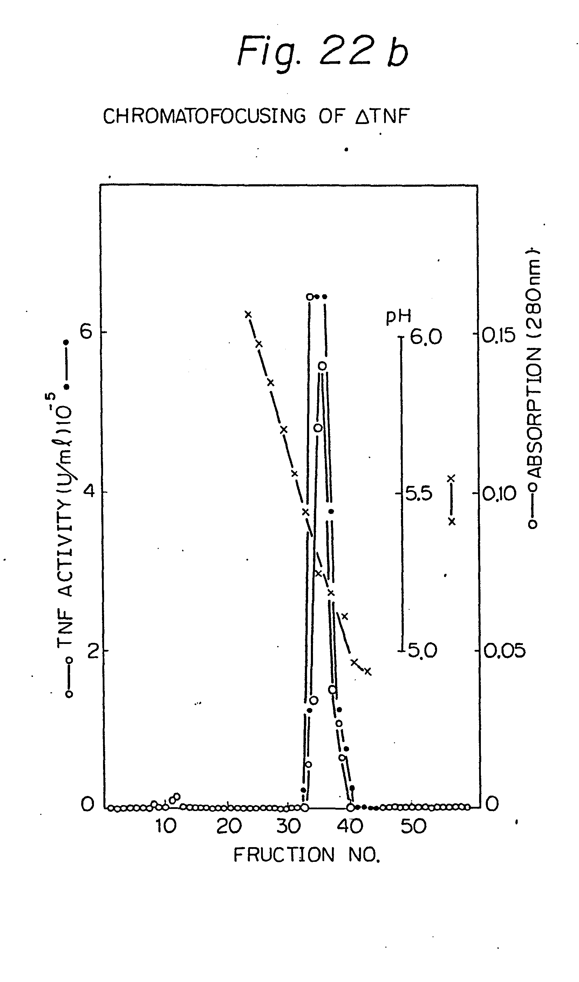

Figs. 22-a and 22-b show the results of chromatofocusing performed on the two peptides characterized in Fig. 21, wherein Fig. 22-a relates to the polypeptide, Recombinant TNF, made of 157 amino acids, and Fig. 22-b refers to the polypeptide of the present invention, Recombinant ΔTNF.

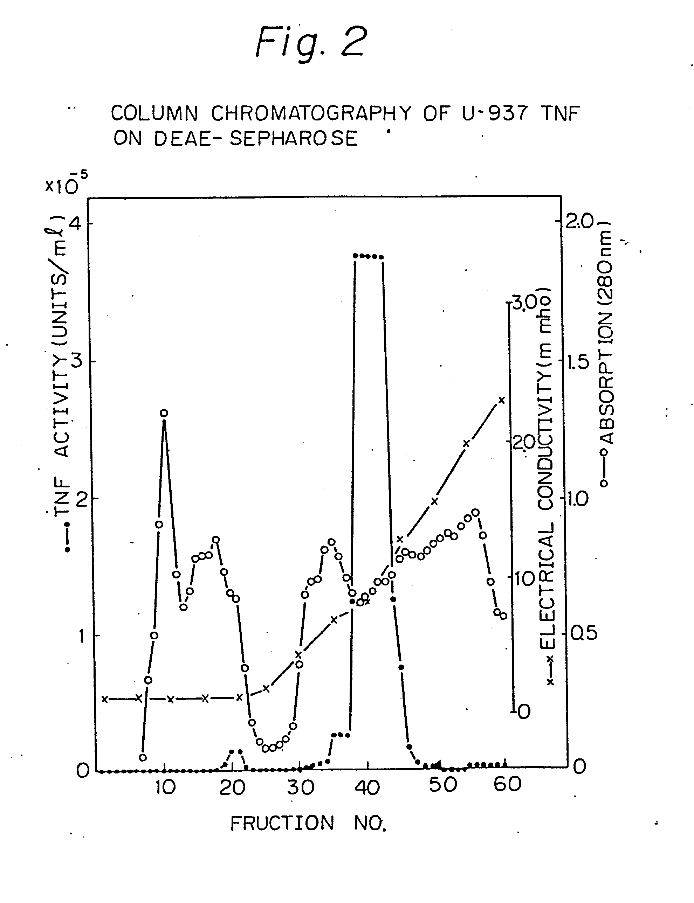

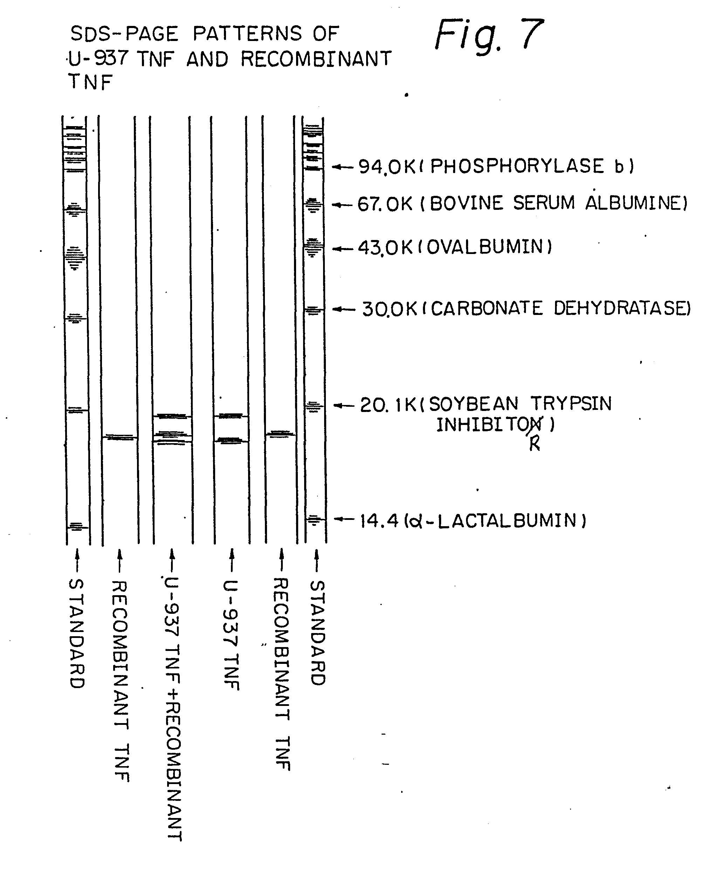

The TNF producing cell used in the present invention was macrophage-like cell U-937 (American Type Culture Collection, CRL 1593) derived from human histiocytic lymphoma. A substance having TNF activity was induced by adding the usual 4β-phorbol 12β-myristate 13α-acetate (PMA), plus retinoic acid and insulin to a culture broth of U-937. The ability of U-937 to produce TNF is described in Camerson, D. J.; Reticuloendothel. Soc., 34, 45 - 52, 1983. The culture supernatant was concentrated about 100-fold in a hollow fibers. The concentrate was subjected to a purification scheme consisting of chromatography on a dye adsorption affinity carrier combined in a proper order with two or more of column chromatography on anion exchange carrier (e.g. DEAE Sepharose), chromatofocusing, column chromatography on hydrophobic carrier (e.g. phenyl Sepharose) and gel permeation chromatography (on, e.g. Sephacryl S-200). For the records obtained in respective purification steps, see Figs. 2 to 6. In analyses by SDS PAGE and silver staining, the purified product exhibited two protein bands corresponding to molecular weights of about 19,000 and 17,000, and the protein corresponding to MW of about 17,000 was the novel polypeptide having TNF activity in accordance with the present invention (see Fig. 7). By gel permeation chromatography on Sephacryl S-200, the polypeptide in native form was found to have a molecular weight of 47,000 ± 5,000, and this taken with the results of SDS PAGE suggests that the polypeptide may occur naturally as an oligomer. Chromatofocusing with a purified sample showed that the native polypeptide of the present invention was between 5.2 and 5.3. The sequence of the first 23 N-terminal amino acid residues in the predominant polypeptide having a molecular weight of about 17,000 as determined by SDS PAGE was Ser-Arg-Thr-Pro-Ser-Asp-Lys-Pro-Val-Ala-His-Val-Val-Ala-Asn-Pro-Gln-Ala-Glu-Gly-Gln-Leu-Gln. This N-terminal amino acid sequence is the same as that starting at the fifth amino acid from the N terminus of the polypeptide reported in Pennica, D. et al. ibid and Wang, A. M. et al. ibid, as consisting of 157 amino acids, and is also the same as the sequence starting at the third amino acid from the N terminus of the polypeptide reported in Shirai, T. et al. ibid, as consisting of 155 amino acids in cDNA clone (see Fig. 1). However, the polypeptide discovered in the present invention is an entirely new type of substance produced by activated macrophage-like cells that could not be anticipated from the references listed above and any of the other prior art references. This substance has a TNF activity comparable to or higher than that of the known polypeptide consisting of 157 or 155 amino acids (maximum cytocidal activity against the L-929 cell to be defined hereinafter: 3.3 x 10$^7$ U/mg protein, see Table 2 below), as well as high heat and pH stabilities. If it is correct to assume that the polypeptide of the present invention was produced by some proteolytic action exerted in the organism on the precursor protein of the type shown in Fig. 1, the polypeptide of the invention composed of 153 amino acids is potentially more stable than the polypeptide composed of 157 or 155 amino acids.

The polypeptide of the present invention has the properties summarized in Table 1 below. In the Examples that follow of present invention, U-937 histiocytic lymphoma cell line was used as the macrophage-like cell, but it should be understood that any other macrophage-like cells such as HL-60, THP-1 and Mono-1-207 may also be used.

### Table 1: Properties of the Polypeptide of the invention

1. Molecular weight as determined by gel permeation chromatography on Sephacryl S-200: 　　　　　　47,000 ± 5,000

2. Molecular weight as determined by SDS PAGE: 　　　　　　ca. 17,000 ± 1,500

3. The first 23-N-terminal amino acid sequence:

　　　1　　　　　　　　　　　5
　　Ser - Arg - Thr - Pro - Ser - Asp - Lys - Pro - Val -

　　　10　　　　　　　　　　15
　　Ala - His - Val - Val - Ala - Asn - Pro - Gln - Ala -

　　　　　20
　　Glu - Gly - Gln - Leu - Gln

4. Isoelectric point as determined by chromatofocusing with PBE 94 as carrier: 　　　　　　5.2 - 5.3

5. Cytocidal activity against L929 cells: 　　　　　　$1.0 - 5.0 \times 10^{7}$ U/mg protein

6. Cross reaction against human TNF antibody: 　　　　　　positive

7. Adsorption on dye adsorption affinity carrier: 　　　　　　positive

On the other hand, the polypeptide of this invention can be produced by the genetic engineering method described hereunder.

A transformant for producing novel polypeptide $^{\Delta N4}$TNF having antitumor activities may be obtained by transforming E. coli with a plasmid variously improved as described below from plasmid pBR322-PL-T4-hTNF (E. coli C600 strain into which the plasmid was integrated was deposited by Deposit No. DSM 3175 to the Culture Collection of the Deutsche Sammlung von Mikroorgamismen) to which was integrated a gene coding for a polypeptide (refer to Fig. 1: abbreviated as TNF hereinafter) consisting of 157 amino acids and having TNF activities.

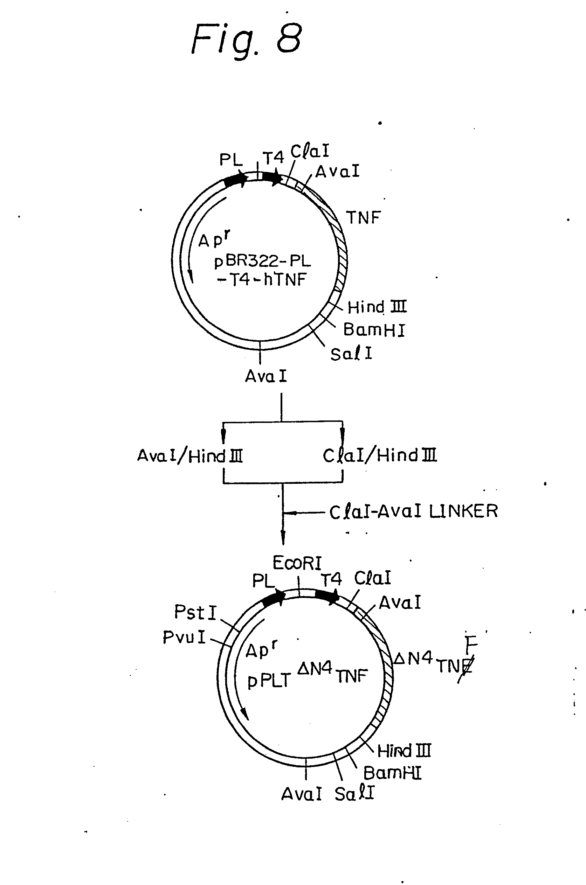

First, plasmid pPLT4$^{\Delta N4}$TNF in which a gene coding for TNF is replaced by a gene coding for $^{\Delta N4}$TNF is constructed by replacing an upstream region of a gene coding for TNF in plasmid pBR322-PL-T4-hTNF by a chemically synthesized DNA linker (refer to Fig. 8). The plasmid pBR322-PL-T4-hTNF contains a DNA fragment constructed so as to express the TNF gene under the control of PL promoter derived from λ phage or a promoter derived from T4 phage. Also, the ribosome binding sequence (or Shine-Dalgarno sequence or SD sequence) used is derived from T4 phage. E. coli transformed by the plasmid also produces $^{\Delta N4}$TNF, but the plasmid is further improved as follows so as to produce $^{\Delta N4}$TNF in much better efficiencies. In the invention, a cDNA fragment from mRNA which is produced from an activated human macrophage-like cell is used as a $^{\Delta N4}$TNF gene, but said gene should not be limited to the cDNA fragment and a chemically synthesized DNA corresponding to the amino acid sequence of $^{\Delta N4}$TNF may also be used.

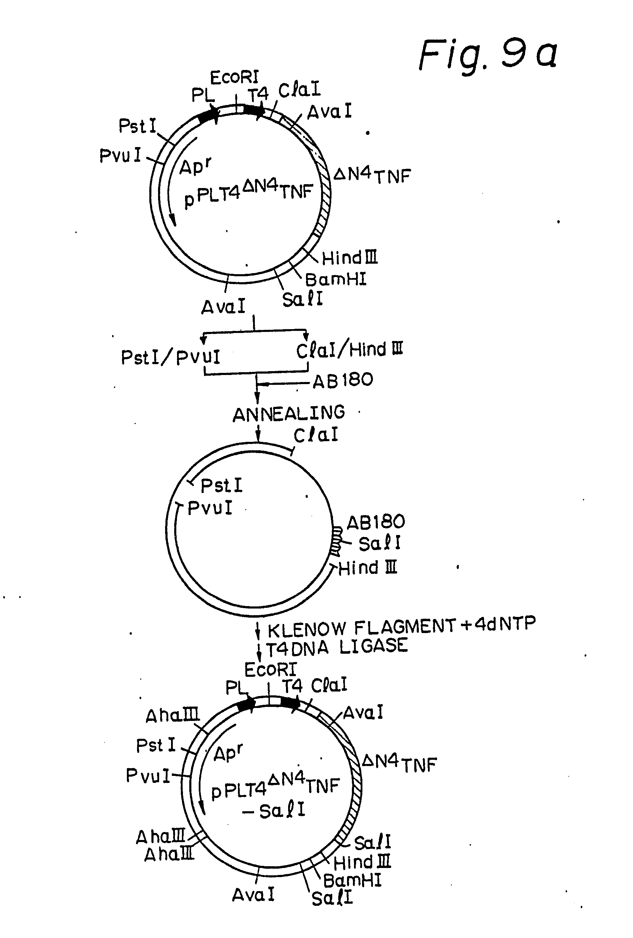

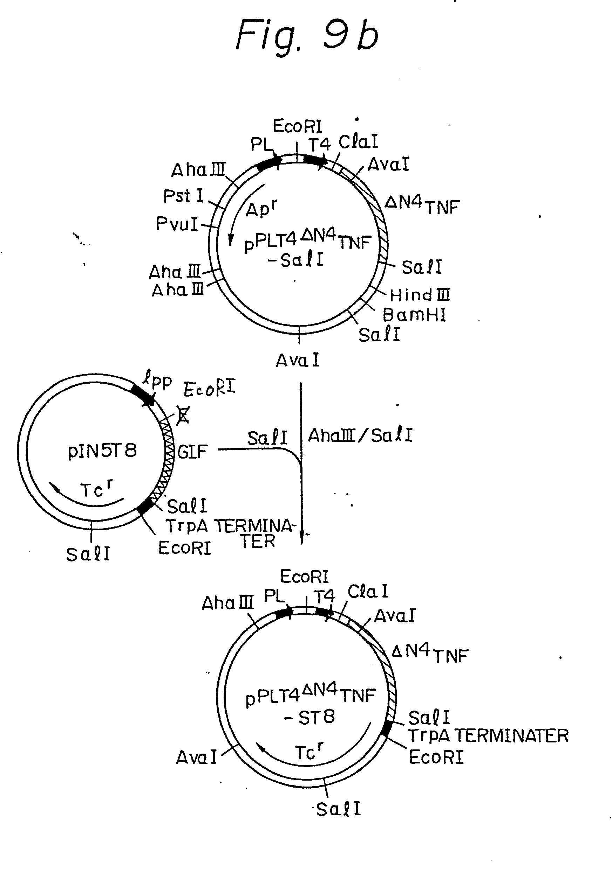

The inventors disclosed in Japanese Patent Application No. 253423/84 that if an alien polypeptide is produced in E. coli, said alien polypeptide may be produced in high yields by joining a transcription terminator just downstream a translation termination codon in a gene coding for said polypeptide. On the basis of the findings, the inventors inserted a terminator derived from an E. coli gene just downstream the translation termination codon of the $^{\Delta N4}$TNF gene. This terminator was derived from plasmid pIN5T8, and inserted as shown in Fig. 9-a and Fig. 9-b. This plasmid is one in which TrpA terminator of E. coli is inserted downstream of a translation termination codon of an exogenous gene in the plasmid pIN5GIF54, and the construction thereof is disclosed by Japanese Patent Disclosure No. 24187/85. That is, a cutting site with restriction endonuclease SalI is introduced downstream the $^{\Delta N4}$TNF gene in plasmid pPLT4$^{\Delta N4}$TNF according to the _in vitro_ mutation method by Morinaga, Y. et al. "Biotechnology" 2, 636 - 639, 1984 (pPLT4$^{\Delta N4}$TNF-SalI in Fig. 9), and a DNA fragment (SalI fragment) containing trpA terminator of pIN5T8 is inserted at SalI cutting site so as to construct pPLT4$^{\Delta N4}$TNFST8 in which a transcription terminator is joined at a site just downstream the translation termination codon of the $^{\Delta N4}$TNF gene. In the process an ampicillin-resistant marker (Ap$^r$) on plasmid pPLT4$^{\Delta N4}$TNF is changed to a tetracycline-resistant marker (Tc$^r$). The $^{\Delta N4}$TNF productivity of _E. coli_ transformed by this plasmid is much greater than that of _E. coli_ transformed by pPLT4$^{\Delta N4}$TNF. Also, host _E. coli_ to be transformed by the $^{\Delta N4}$TNF-expression plasmid using the PLT4 promoter should be _E. coli_ transformed beforehand by a plasmid (e.g., pCI857) which is capable of expressing a temperature-sensitive repressor gene (e.g., CI857) of λ phage and also compatible with other plasmids such as pBR322, or bacteria lysogenic to λ phage and having the above repressor gene. The reason that the host capable of expressing the temperature-sensitive repressor gene or λ phage is used is because the PL promoter is operated stably and efficiently to produce the desired polypeptide in high yields. Plasmid pCI857 which bears temperature-sensitive repressor gene CI857 of λ phage is described in Gene 22, 103 - 113, 1983 (Remand, E., Tsao, H. & Fiers, W.), and the plasmid has a kanamycin-resistant gene integrated and is compatible with a plasmid derived from pBR322. _E. coli_ WA802 and W3110 transformed by this plasmid are represented by WA802/CI and WA3110/CI, respectively.

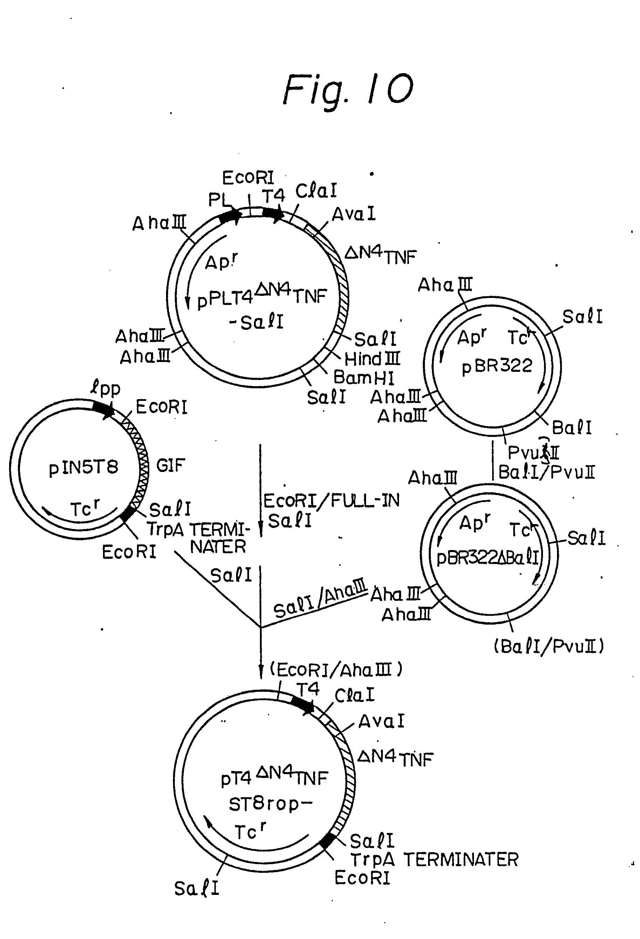

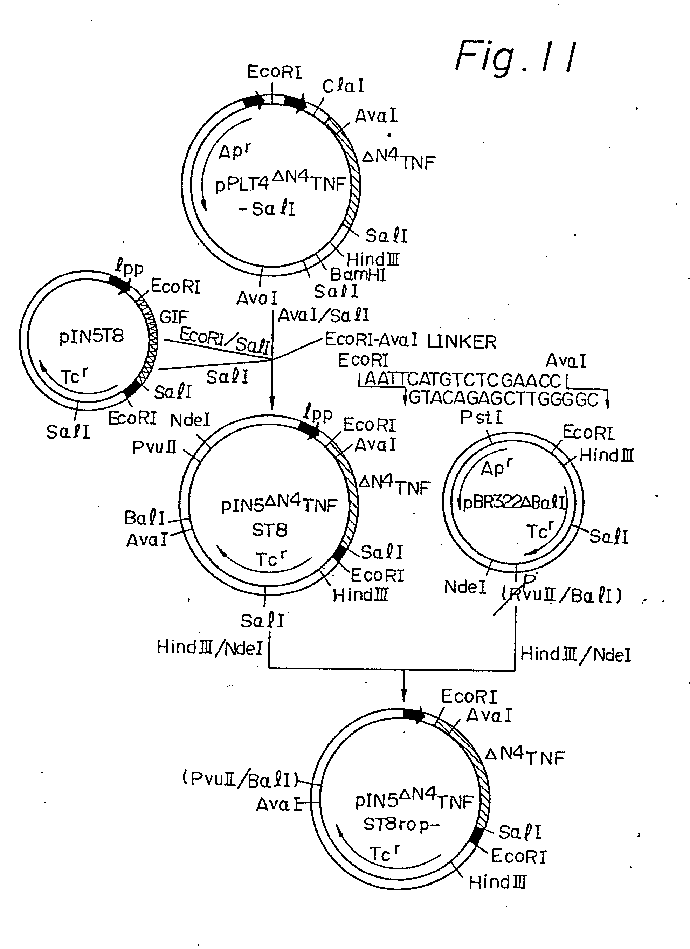

Plasmid pPLT4$^{\Delta N4}$TNFST8 obtained above is considered to express to $^{\Delta N4}$TNF gene under the control of PL promoter derived from λ phage and a promoter derived from T4 phage. The inventors further carried out elimination of the rop region (repressor of primer: which has activities inhibiting the synthesis of primer RNA essential to replication of plasmid DNA: Proc. Natl. Acad. Sci. USA vol. 79, pp 6313 - 6317, 1982) which is considered to control modification of promoter region and the number of copies of plasmid to construct a plasmid for expression $^{\Delta N4}$TNF in high yields (refer to Fig. 10 and Fig. 11).

Modification of expression system under the control both of PL promoter and T4 promoter to that of T4 promoter alone (Fig. 10) is carried out in order to generalize the host E. coli and to produce the desired polypeptide constitutively. If PL promoter is used, CI gene which is a repressor of λ phage should necessarily be present in the host cell. Therefore, restrictions on the host E. coli are reduced by use of T4 promoter alone. Fig. 10 shows plasmid pT4$^{\Delta N4}$TNFST8$^{rop-}$ constructed from plasmid pPLT4$^{\Delta N4}$TNF-SalI (see Fig. 9-a) by modification of the promoter region (PL-T4 → T4), elimination of the rop region and modification of the chemical resistant marker (Ap$^r$ → Tc$^r$), and addition of TrpA terminator. Modification of the promoter region and the drug resistant marker and elimination of the rop region are carried out by eliminating an EcoRI-SalI fragment containing the PL promoter region from pPLT4$^{\Delta N4}$TNF-SalI and inserting a SalI-AhaIII fragment containing a tetracycline resistant gene (Tc$^r$) of plasmid pBR322 BalI which was derived from pBR322 and constructed by eliminating 622 base pairs between BalI and PvuII cutting sites from pBR322. Also, addition of the transcription terminator is carried out by inserting a SalI fragment containing the TrpA terminator of the above-mentioned plasmid pIN5T8 into the SalI cutting site at the terminal region of the $^{\Delta N4}$TNF gene in pPLT4$^{\Delta N4}$TNF-SalI. E. coli transformed by plasmid pT4$^{\Delta N4}$TNFST8$^{rop-}$ thus obtained can produce $^{\Delta N4}$TNF in high yields. The ribosome binding sequence between the promoter region and the $^{\Delta N4}$TNF gene in the above plasmids (pPLT4$^{\Delta N4}$TNFST8 and pT4$^{\Delta N4}$TNFST8$^{rop-}$) has the SD sequence derived from T4 phage.

The promoter region and the SD sequence should not be limited to the above-mentioned ones, and any one suitable for the host used may be used. For example, if E. coli is used for the host, a promoter region for the outer cell membrane lipoprotein gene (lpp) of E. coli may be used for the promoter region, and chemically synthesized

0205038

oligonucleotide (DNA) fragment which is designed so as to be complementary with 3' terminus of 16S ribosome RNA of E. coli may be used for the SD sequence. Fig. 11 shows a process for constructing plasmid pIN5$^{\Delta N4}$TNFST8$^{rop-}$ which expresses the $^{\Delta N4}$TNF gene under the control of lpp promoter and the synthesized SD sequence by use of plasmid pIN5T8 already constructed as described above and an EcoRI - AvaI linker. In this pIN5T8 plasmid, human γ-interferon gene (GIF) is inserted into the plasmid so as to express GIF under the control of lpp promoter and chemically synthesized SD sequence. First, a small fragment ClaI-AvaI containing a region at the 5'-terminal region of $^{\Delta N4}$TNF in pPLT4$^{\Delta N4}$TNF-SalI is eliminated, and a chemically synthesized EcoRI-AvaI linker,

$$5' \; |AATTCATGTCTCGAACC|\_ \; 3'$$
$$\phantom{5' \; |}\lfloor GTACAGAGCTTGGGGC \downarrow \; T$$
$$\phantom{5' \; |}(EcoRI) \qquad\qquad (AvaI)$$

is joined. Then EcoRI-SalI fragment containing the lpp promoter, chemically synthesized SD sequence and a Tc$^{r}$ gene of pIN5T8 is introduced so as to construct plasmid pIN5$^{\Delta N4}$TNFST in which $^{\Delta N4}$TNF gene is joined downstream of the lpp promoter and the chemically synthesized SD sequence and a transcription terminator is joined just downstream of the translation termination codon of the $^{\Delta N4}$TNF gene. $^{\Delta N4}$TNF may be produced by use of the plasmid, a small fragment PvuII-BalI is eliminated from this plasmid to construct plasmid pIN5$^{\Delta N4}$TNFST8$^{rop-}$. E. coli transformed by the plasmid produces $^{\Delta N4}$TNF almost equivalent to that produced by E. coli transformed by plasmids pPLT4$^{\Delta N4}$TNFST8 and pT4$^{\Delta N4}$TNFST8$^{rop-}$.

Host E. coli (W3110, W3110/CI, WA802 or WA802/CI) transformed by various types of plasmids described above are cultured in an appropriate medium by shaking culture or aeration/agitation culture. The bacterial cells obtained are disrupted by the freeze-thawing method by use of lysozyme or by means of a French press, a homogenizer, etc. The extract from the disrupted bacterial cells is subjected to SDS polyacrylamide gel electrophoresis (abbreviated as SDS PAGE hereinafter) and measurements of TNF activities to check the amount of $^{\Delta N4}$TNF produced (see Table 3 and Table 4). The TNF activities are determined according to a method described in Lymphokines, vol. 2, 235, Academic Press (1981) as will be described later. Also, $^{\Delta N4}$TNF contained in the extract from the culture solution of the transformed E. coli strain is purified according to the method described in the present specification, and is then subjected to amino acid analysis. The amino acid composition of $^{\Delta N4}$TNF thus obtained by the process of the invention is consistent with the composition expected (see Table 6). Thus, the process of this invention was confirmed to be useful for producing polypeptides from the culture solution described above.

The polypeptide purified in the invention is different from the polypeptide consisting of 157 amino acids shown in Fig. 1 which has already been reported in terms of physical and chemical properties and TNF activities (specific activities). The 157 amino acid polypeptides may be obtained by culturing E. coli transformed by plasmid pBR322-PL-T4-hTNF shown in Fig. 8 followed by purification of the culture bacteria according to a method similar to that described above. The polypeptide produced by this invention has TNF activities higher than those of the polypeptide shown in Fig. 1.

The present invention also provides a novel method for TNF purification making use of the ability of the polypeptide described above to be adsorbed on a dye adsorption affinity carrier. Mouse and rabbit TNFs have been known to lack the ability to be adsorbed on Cibachrone Blue 3GA dye adsorption affinity carrier (Tamura & Ishida, Jpn. J. Cancer Chemother., 11, 1369 - 1378, 1984). Since TNF generally has no specificity to species, it may well be predicted that human TNF also lacks the ability to be adsorbed on Cibachrone Blue 3GA dye adsorption affinity carrier. Unexpectedly, however, the present inventors have found that the polypeptide of the present invention having TNF activity could be adsorbed on this particular carrier and be subsequently eluted by increased salt concentrations or pHs. This fact indicates the possibility of using chromatography on a dye adsorption affinity carrier for the purpose of purifying a polypeptide with TNF activity, and as a matter of fact, this technique proved very effective in purifying the polypeptide of the present invention. No case has been reported of using a dye adsorption affinity carrier in purification of a polypeptide having human TNF activity. Another advantage of performing chromatography on this carrier is that the heat treatment step that may involve the denaturation of the desired polypeptide (Shirai et al., ibid, and unexamined Published Japanese Patent Application No. 19719/1985). The method of purification in accordance with the present invention may also be used in obtaining a purified substance with TNF activity from a culture of a transformant capable of producing TNF activity substance created by the recombinant DNA technology.

In the Examples shown later in this specification, Matrex Blue A (Amicon) was used as a dye adsorption affinity carrier, but Matrex Red A (Amicon) and Blue Trisacryl (LKB) may also be used.

As described above, the polypeptide of the present invention can be obtained by purifying a culture supernatant of macrophage-like cells. It may also be obtained as described above by culturing microorganism or animal cells that have been transformed by a plasmid or phage vector incorporating DNA coding for this peptide (which may be either cDNA prepared from mRNA produced by said macrophage-like cells or a chemically synthesized DNA) in such a manner that it can be expressed under the control of a suitable promoter. The polypeptide prepared by the recombinant DNA technology (peptide with TNF activity composed of 153 amino acids: $^{\Delta N4}$TNF) is indistinguishable from the polypeptide isolated from the culture of U-937 in terms of molecular weight as estimated for monomeric form by SDS PAGE, isoelectric point as determined by chromatofocusing, in vitro antitumor activity, heat stability, HPLC analysis, reactivity with TNF antibody, affinity for various carriers used in chromatographic purification techniques, pH stability, $NH_2$-terminal amino acid sequence, etc. Said $^{\Delta N4}$TNF is

comparable to or higher than the polypeptide consisting of 157 amino acids (Pennica, D. et al., ibid and Wang, A. M. et al., ibid) in terms of in vitro antitumor activity, as well as heat and pH stability. The two TNFs also differ with respect to other properties.

The present invention is hereunder described in greater detail by reference to working examples.

Example 1

1. Induction and production of TNF active substance from U-937

Human macrophage-like cells U-937 were cultured in a roller bottle (Falcon) at 37°C for 2 days using 500 ml of an RPMI 1640 medium (GIBCO) containing 0.22% sodium hydrogen-carbonate ($NaHCO_3$), 60 µg/ml of Kanamycin, 0.03% glutamine, 5% of fetal bovine serum (GIBCO) and 5% of horse serum (GIBCO) after subjecting said medium to an initial pH of 7.2 - 7.5 by $CO_2$ charge. Thereafter, the culture was centrifugally washed with RPMI 1640 containing 0.22% $NaHCO_3$, 60 µg/ml of kanamycin and 0.03% glutamine. About $3 \times 10^9$ U-937 cells were suspended in 2.7 liters of RPMI 1640 medium containing 0.22% $NaHCO_3$ and 0.03% glutamine. An 8-liter spinner flask (Bellco) was used as an incubator for induction and production of the desired substance having TNF activity. To the suspension of U-937 cells, 300 ml of an RPMI 1640 medium containing 320 nM of PMA, 500 nM of retinoic acid and 300 units of bovine insulin (all of them were products of Sigma), as well as 0.22% $NaHCO_3$ and 0.03% glutamine was added so that the final concentrations of U-937 cells, PMA, retinoic acid and bovine insulin were respectively about $1 \times 10^6$/ml, 32 nM, 50 nM and 100 U/1,000 ml. After incubation at 37°C for 24 hours in the dark at 120 rpm, the culture was centrifuged to provide a supernatant in preparation for the measurement of TNF activity.

2. Measurement of TNF activity

TNF activity was determined by measuring the in vitro tumor cell killing activity in accordance with the following method which was a modification of the method described by Ruff, M. R. and Gifford, G. E. (1981) in Lymphokine Reports (Pick, E., ed) Vol. 2, pp. 235 - 272, Academic Press, New York.

One tenth of a milliliter of cell suspension in Eagle's MEM plus 10% fetal bovine serum, 0.1% $NaHCO_3$ and 0.03% glutamine (6 x $10^5$ L-929 cells in log growth stage per ml which were deposited at ATCC as CCLI and which were subspecies of mouse L cells) was added to each of the 96 wells in a flat bottom tissue culture microplate (Nunc) for 0.1 ml of the supernatant sample that had been serially diluted in the same Eagle's MEM. To each well, Actinomycin D (Makor Chemicals) had been added to a final concentration of 1 μg/ml. The microplate was incubated at $37^{\circ}C$ for ca. 18 hours in air containing 5% $CO_2$. After incubation, the supernatant was discarded and 0.1 ml of a 0.5% aqueous Crystal Violet solution containing 20% methanol, so as to stain adherent L-929 cells for 15 minutes at room temperature. The stained microplate was thoroughly washed with water. After drying, 0.1 ml of 33% aqueous acetic acid solution was added to extract the dye from the stained cell lysate. The absorbance of the extract at 577 nm was measured with Titertek Multiskan Spectrophotometer. The reciprocal of the dilution for the sample having an absorption corresponding to 50% of the value for the control was used as the TNF activity of the sample and indicated in units/ml (U/ml). At least four measurements were made for each sample and their average was taken.

3. Purification of substance having TNF activity

After concentrating the culture supernatant (obtained in Example 1-1 through a hollow fiber HIP 10-20 (Amicon), the substance having TNF activity present in the supernatant (this substance is hereunder simply referred to as TNF) was purified by the following scheme consisting, in sequence, of column chromatography on DEAE Sepharose, chromatofocusing, column chromatography on Phenyl-Sepharose and chromatography on dye adsorption affinity carrier. It should however be noted that this is not the only order of the purifying steps that can be used in the present invention.

a) DEAE-Sepharose column chromatography (see Fig. 2)

Twenty-five liters of the culture supernatant of U-937 cells obtained in Example 1-1 was subjected to cyclic concentration (to 130 ml) and buffer replacement by an ultrafiltration unit DC-3 (Amicon) using a hollow fiber HIP 10-20. The volume of the final concentrate was 250 ml.

The concentrate was loaded onto an anion exchanger, DEAE-Sepharose CL 6B (Pharmacia, 2.5 $cm^6$ x 16 cm) that had been thoroughly equilibrated with 10 mM tris-HCl buffer (pH: 7.4). After thoroughly washing the column with the same buffer, TNF was eluted with aqueous sodium chloride having a linear concentration gradient of 0 - 0.2 M in the same buffer. Elution patterns of protein and TNF activity are shown in Fig. 2. A single peak of TNF activity was observed in the neighborhood of 0.08 M NaCl.

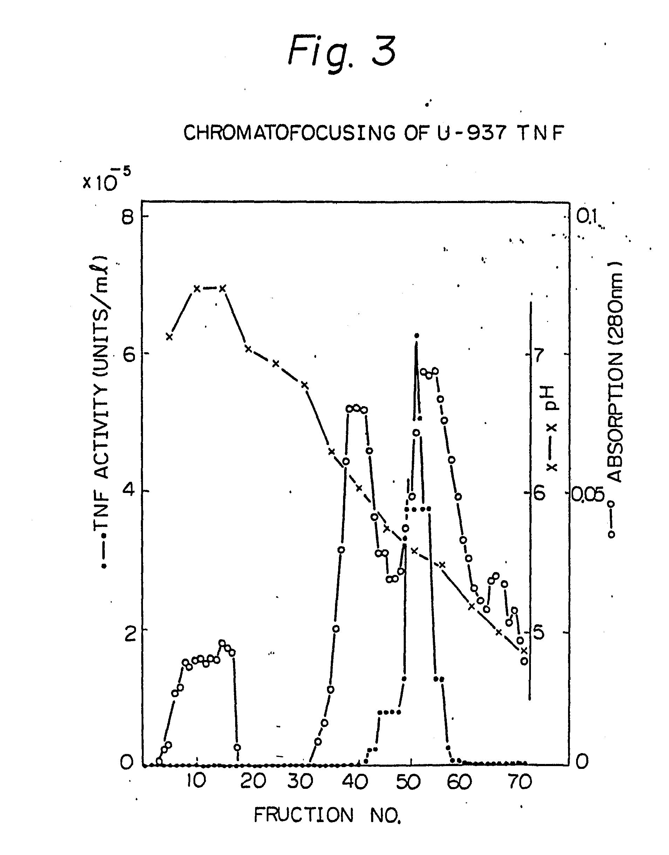

b) Chromatofocusing (Fig. 3)

Eighty ml of the active fraction was dialyzed against 2 liters of 25 mM imidazole HCl buffer (pH: 7.0) and loaded onto chromatofocusing carrier column PBE 94 (Pharmacia, 1.0 $cm^6$ x 40 cm) that had been thoroughly equilibrated with 25 mM imidazole HCl (pH: 7.0). Following adjustment to pH of 4.0 with HCl, TNF was eluted with pH gradient formed by causing a 10-fold diluted polybuffer 74 (Pharmacia) to flow through the column. The resulting elution patterns are shown in Fig. 3. The TNF activity was eluted as a single peak at pHs in the neighborhood of 5.5 $\pm$ 0.3. Since chromatofocusing performed after the purification effected by chromatography on dye adsorption affinity carrier in subsequent step d) resulted in elution at pHs of 5.2 - 5.3, the polypeptide of the present invention was found to have an isoelectric point of 5.2 - 5.3.

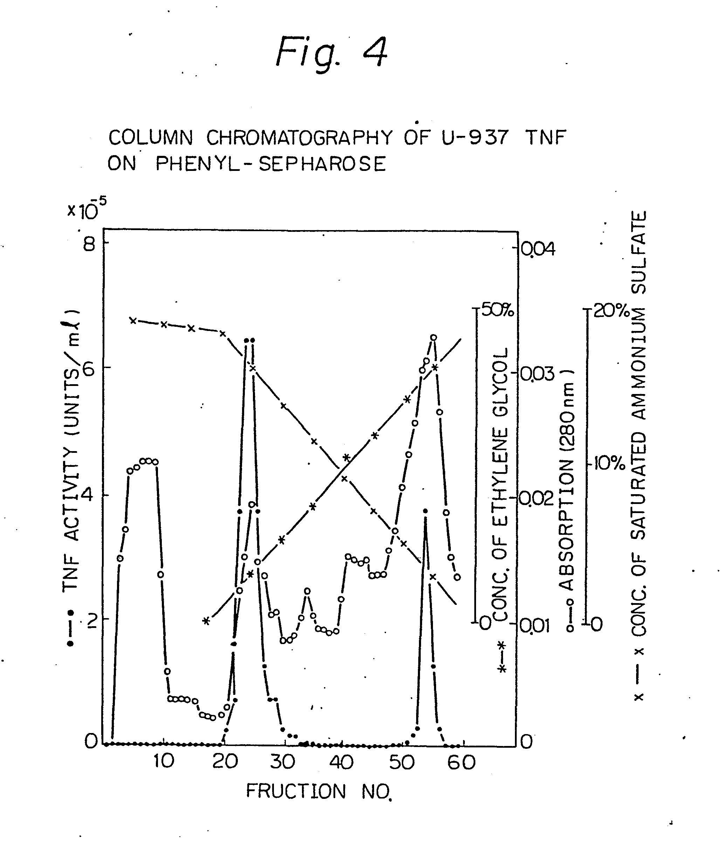

c) Phenyl-Sepharose column chromatography (see Fig. 4)

Fifty ml of the active fraction was mixed with ammonium sulfate to give a saturated concentration of 20%. The mixture was loaded onto a hydrophobic carrier, Phenyl-Sepharose CL 4B column (Pharmacia, 1.0 $cm^6$ x 10 cm) that had been thoroughly equilibrated with 10 mM tris-HCl buffer (pH: 7.4) plus 20% saturated ammonium sulfate. The adsorbed TNF was eluted by simultaneous density gradients of 20% (saturation) to 0% of ammonium sulfate and 0% to 50% of ethylene glycol in the same buffer. As shown in Fig. 4, the peak having TNF activity was divided into two fractions. The relative values of TNF activities in the two fractions varied with the amount of protein loaded onto the column, and when each fraction was subjected to another run of chromatography, the TNF activity was again divided into two fractions. Column chromatography on Phenyl-Sepharose, SDS PAGE analysis of the two fractions, and amino acid analysis (to be described later) suggested that these results were not due to the presence of two different types of TNF but probably due to the variance in the interaction between TNF and carrier resin resulting from the difference in the state of TNF molecule at the time of addition of ammonium sulfate.

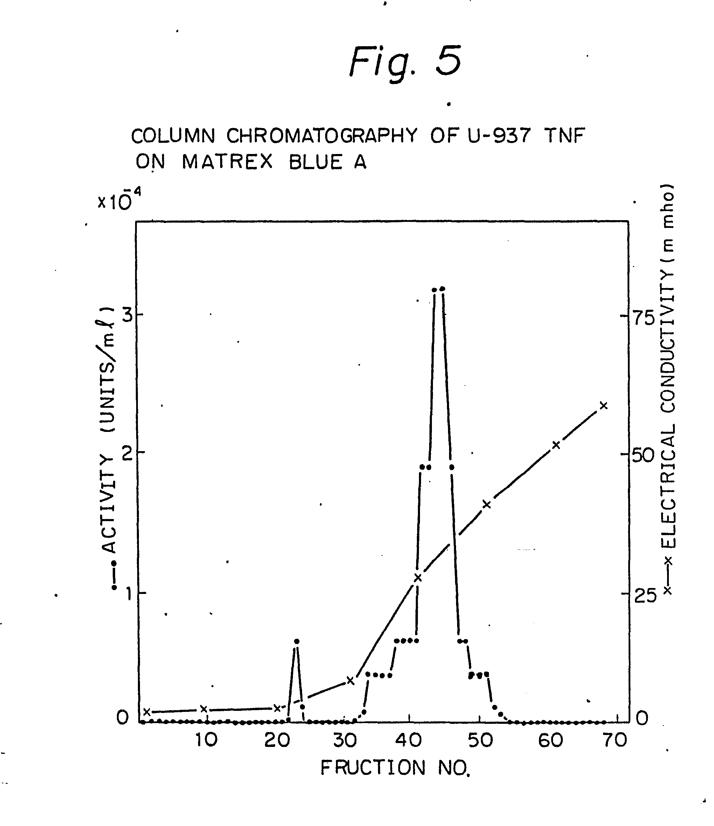

d)   Chromatography on dye adsorption affinity carrier (see Fig. 5)

Matrex Blue A (Amicon, 1.0 cm$^6$ x 7 cm) was used as the dye adsorption affinity carrier. Of the two fractions having TNF activity that were obtained in c), the fraction having higher TNF activity (hereunder referred to as Fraction 1) weighing 75 ml was thoroughly dialyzed against 10 mM tris-HCl buffer (pH: 7.4) and loaded onto the Matrex Blue A column equilibrated with the same buffer, followed by TNF elution with aqueous NaCl having a linear concentration gradient of 0 - 1.2 M in the same buffer. As a result, the TNF activity pattern shown in Fig. 5 was obtained. The same elution pattern was obtained for the fraction having lower TNF activity (hereunder Fraction 2). These results show the effectiveness of the dye adsorption affinity carrier in TNF purification.

The TNF activity, amount of protein and other analytical data obtained in each of the purification steps a) to d) are summarized in Table 2. The amount of protein was determined by the method of Bradford, M. M. (Anal. Biochem. 72, 248 - 254, 1976) using coupling with Coomassie brilliant blue G-250.

## Table 2

### Purification of TNF produced by U-937

| Purification step | Volume (ml) | Activity (U/ml)$\times 10^{-4}$ | Protein (mg/ml) | Total activity (U)$\times 10^{-4}$ | Specific activity (U/mg)$\times 10^{-4}$ | Degree of purification | Recovery yield (%) |
|---|---|---|---|---|---|---|---|
| unpurified supernatant | 250 | 15 | 0.74 | 3750 | 20.3 | 1 | 100 |
| DEAE-Sepharose column chromatography | 80 | 44 | 0.27 | 3520 | 163 | 8 | 94 |
| Chromatofocusing | 50 | 44 | 0.066 | 2200 | 667 | 33 | 59 |
| Phenyl-Sepharose column chromatography | 75 | 10 | - | 750 | - | - | 20 |
| Matrex Blue A column chromatography | 90 | 3.3 | 0.0010 | 297 | 3300 | 162 | 8 |

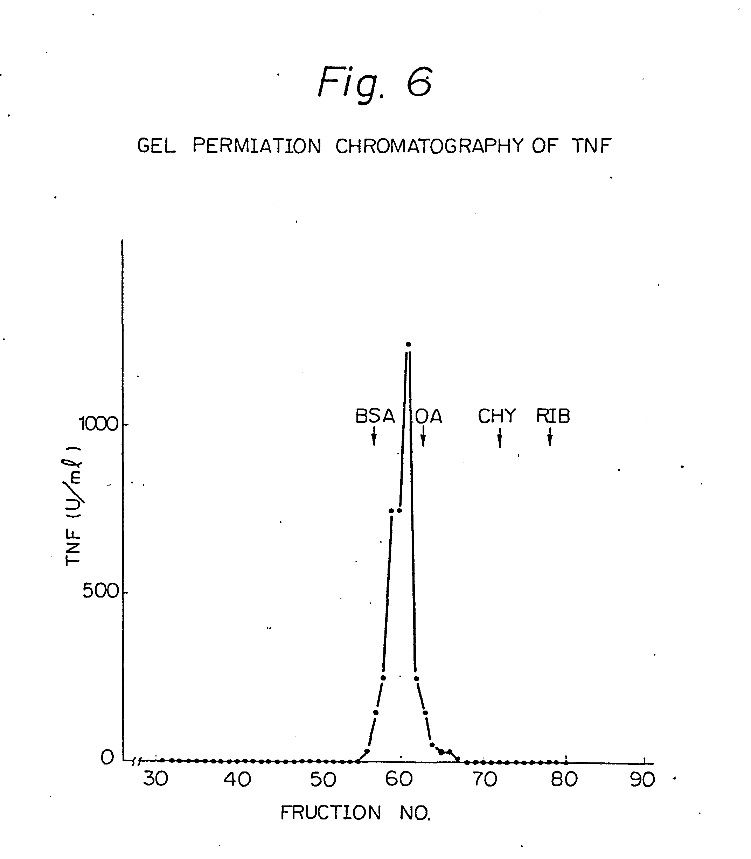

4.  <u>Measurement of molecular weight (see Fig. 6)</u>

Five ml of the active fraction obtained in 3-d) was loaded onto a gel permeation carrier, Sephacryl S-200 (Pharmacia, 2.5 cm$^{\emptyset}$ x 100 cm) and the molecular weight of naturally occurring TNF was estimated by comparison with standard proteins.  More specifically, the active fraction was loaded onto the Sephacryl S-200 column that had been thoroughly equilibrated with 10 mM tris-HCl buffer (pH: 7.4) and 0.5 M NaCl.  The resulting elution pattern for the active pattern is shown in Fig. 6.  On the basis of comparison with standard proteins, i.e., bovine serum albumin (BSA, MW 67 K), ovalbumin (OA, MW 45 K), chymotripsinogen A (CHY, MW 25 K) and ribonuclease (RIB, MW 13.7 K), the polypeptide of the present invention was calculated to have a molecular weight of 47,000 ± 5,000 in native form.  As will be described later in this specification, the monomeric form of TNF was found to have a molecular weight of about 17 K (partly 19 K) by SDS PAGE analysis, so it was assumed that the native form of the polypeptide of the present invention had a subunit structure.  Furthermore, two samples, one treated with a reducing agent, 2-mercaptoethanol and the other untreated, exhibited an approximately 17 K protein band by SDS PAGE, and a slice of this band exhibited TNF activity.  In view of these two facts, the native form of the polypeptide of the present invention was assumed to be a homo-oligomer having no S-S crosslink in the subunit structure.

The same elution pattern as shown in Fig. 6 was obtained for Fraction 2 after purification with Matrex Blue A.

Part of each of Fractions 1 and 2 obtained in 3-d) was subjected to SDS PAGE using 13% polyacrylamide gel.  Two protein bands were obtained at molecular weights of about 17,000 and about 19,000 (see Fig. 7).

It was assumed that the amount of protein corresponding to Mr of ca. 17,000 on the gel in Fig. 7 was about 3 - 5 times as great as that of protein corresponding to Mr of ca. 19,000.

-22-     0205038

The polypeptide of the present invention is the one which corresponds to the molecular weight of ca. 17,000, and from the gel band in Fig. 7, the molecular weight of this protein was calculated to be 17,000 ± 1,500. It was confirmed by the Western blotting method that either of the two proteins (19 K and 17 K) cross reacted with any of the antibodies prepared from rabbit sera using as antigens three peptide fragments selected from an amino acid sequence predicted from the TNF cDNA sequence.

However, when a protein purified from an E. coli culture that was constructed by the recombinant DNA technology and which was capable of producing the polypeptide composed of 157 amino acids (see Fig. 1) as described in Pennica, D. et al., ibid (for details of this protein, see Example 2 below) was subjected to SDS PAGE using 13% polyacrylamide gel, it was found that the polypeptide of the present invention (U-937 TNF in Fig. 7) obviously differed from said protein with 157 amino acids (Recombinant TNF in Fig. 7) and had a lower molecular weight. Details of the process for preparing the protein made of 157 amino acids using recombinant E. coli are given in Example 2 hereunder.

5. Determination of NH$_2$-terminal amino acid sequence

Fraction 1 obtained in 3-c) was purified by column chromatography on Matrex Blue A. The purified fraction was subjected to reverse-phase high performance liquid chromatography Type LC-6A (Shimadzu Seisakusho) using Microbondapack C$_{18}$ column (Waters). The concentrated TNF was freeze-dried. Thirty micrograms (1,700 pM) of the freeze-dried product was supplied to gas-phase protein sequencer Model 470 A (Applied Biosystems) and the NH$_2$-terminal amino acid sequence was analyzed by th Edman method.

The sequence of the first 23 NH$_2$-terminal amino acid residues was

```
         1                        5
     Ser - Arg - Thr - Pro - Ser - Asp - Lys - Pro -
            10                        15
     Val - Ala - His - Val - Val - Ala - Asn - Pro -
                        20
     Gln - Ala - Glu - Gly - Gln - Leu - Gln
```

The same results were obtained for TNF present in Fraction 2 by following the same analytical procedures.

6. Heat and pH stability

The polypeptide of the present invention was checked for its heat and pH stability by using the hollow fiber concentrate of culture obtained in 3-a) (protein: 0.74 mg/ml, TNF: ca. 0.6%). As shown in Fig. 15-a, the TNF in the concentrate remained stable up to 65°C for a period of 30 minutes and was completely inactivated at 85°C. Another sample of the concentrate was dialyzed against adequate amounts of buffer with varying pHs for 14 hours at 4°C, and subsequently dialyzed against 10 mM tris-HCl buffer (pH: 7.5) for 1 hour at 4°C. The residual TNF activity data are shown in Fig. 16-a, from which one can see that the TNF in the concentrate remained stable over the pH range of 4.9 - 10.7.

Example 2

1. Construction of a plasmid for producing $^{\Delta N4}$TNF

a) Preparation of a $^{\Delta N4}$TNF-expression plasmid by use of PLT4 promoter (refer to Fig. 8 and Fig. 9)

Plasmid pBR322-PL-T4-hTNF which was separated from E. coli deposited under No. DSM 3175 was completely digested by use of AvaI and HindIII to separate a DNA fragment containing the most part of the TNF gene. On the other hand, pBR322-PL-T4-hTNF was decomposed by use of ClaI and HindIII to separate a DNA fragment containing a PLT4 promoter and a β-lactamase gene (Ap$^r$). Then, a chemically synthesized linker DNA $\left[\begin{array}{l}\text{5'-CGATACTACTATGTCTCGAACC-3'}\\\text{TATGATGATACAGAGCTTGGGGCT-5'}\end{array}\right]$ by means of a 380A DNA synthesizer by Applied Biosystem Co. which had ClaI and AvaI cohesive ends was mixed with both of the DNA fragments, and they were joined by use of T4DNA ligase to construct pPLT4$^{\Delta N4}$TNF (Fig. 8). Then, a SalI cutting site was introduced just downstream the $^{\Delta N4}$TNF structural gene termination codon according to in vitro modification shown below in order to add an E. coli Trpa transcription terminator just downstream the $^{\Delta N4}$TNF structural gene. Namely, in vitro mutation was carried out by use of chemically synthesized in vitro mutation oligonucleotide AB180 (36-base oligonucleotide consisting of 5'-ATCATTGCCCTGTGAGTCGACCGAACATCCAACCTT-3') and plasmid pPLT4$^{\Delta N4}$TNF according to the method by Y. Morinaga et al. (Morinaga, Y. et al., Biotechnology, 2, 636 - 639, 1984) (Fig. 9-a). That is, pPLT4$^{\Delta N4}$TNF was completely digested by use of PvuI and PstI to separate DNA fragment (I) in which 126 base pairs between the PvuI - PstI cutting sites are eliminated. On the other hand, the same pPLT4$^{\Delta N4}$TNF was cleaved by use of ClaI and HindIII to separate DNA fragment (II) which was free from the TNF gene region (about 0.6 kb). DNA fragments (I) and (II) and 36-base oligonucleotide AB180 were mixed. Denaturation of the double chain DNA was carried out at 100°C and the temperature was slowly lowered to room temperature to carry out double chain formation. At the time, a DNA molecule in which DNA fragments (I) and (II) and oligonucleotide AB180 are annealed was formed. To the reaction solution were added dNTP, the Klenow fragment of DNA polymerase, and T4DNA ligase to fill-in the single chain part and to form a complete double chain, ring DNA. E. coli WA802/CI was transformed by use of the reaction solution to obtain an ampicillin-resistant clone. Analysis of the plasmid DNA contained in the ampicillin-resistant clone revealed that the plasmid is pPLT4$^{\Delta N4}$TNF-SalI which has a SalI cutting site just downstream of the $^{\Delta N4}$TNF structural gene (Fig. 9-a).

Then, plasmid pPLT4$^{\Delta N4}$TNFST8, in which a TrpA terminator was introduced just downstream of translation termination codon of the $^{\Delta N4}$TNF gene and a tetracycline-resistant gene (Tc$^r$) was introduced in place of the β-lactamase gene (Ap$^r$), was prepared by used of pPLT4$^{\Delta N4}$TNF-SalI was completely digested by use of AhaIII and SalI to separate a DNA fragment containing the $^{\Delta N4}$TNF structural gene and the PLT4 promoter region and having an AhaIII end and a SalI end, and a DNA fragment (2.6 kb) having an AhaIII end and a SalI end. Also, a DNA fragment (about 0.7 kb) containing a TrpA terminator was obtained by digesting pIN5T8 with SalI. The three DNA fragments were mixed and joined by addition of T4DNA ligase to construct pPLT4$^{\Delta N4}$TNFST8 (Fig. 9-b).

b) <u>Preparation of expression plasmid pT4$^{\Delta N4}$TNFST8$^{rop-}$by</u>
   <u>function of T4 promoter (refer to Fig. 10)</u>

Plasmid pPLT4$^{\Delta N4}$TNF-SalI (refer to Fig. 9-a and Fig. 9-b) prepared in item a was cleaved by use of EcoRI, and the EcoRI cohesive ends were filled-in by use of T4DNA polymerase and 4dNTP. Then, the plasmid was digested by use of SalI to obtain a DNA fragment (about 0.6 kb) containing the T4 promoter and the $^{\Delta N4}$TNF structural gene. Plasmid pIN5T8 was cleaved by use of SalI, and a DNA fragment (about 0.7 kb) containing a TrpA terminator was obtained. On the other hand, plasmid pBR322 was digested by use of BalI and PvuII, and was joined by use of T4DNA ligase to obtain plasmid pBR322$\Delta$BalI in which 622 base pairs between the BalI and PvuII cutting sites have been deleted. Plasmid pBR322 BalI was digested by SalI and AhaIII to separate a DNA fragment (about 2 kb) containing a initiating site for replication. Three types of DNA fragments thus obtained are mixed and joined to each other by use of T4DNA ligase to construct pT4$^{\Delta N4}$TNFST8$^{rop-}$.

c) <u>Preparation of expression plasmid pIN5$^{\Delta N4}$TNFST8$^{rop-}$by</u>
   <u>function of lpp promoter (refer to Fig. 11)</u>

Plasmid pPLT4$^{\Delta N4}$TNF-SalI (refer to Fig. 9-a and Fig. 9-b) was completely digested by use of AvaI and SalI to separate a DNA fragment (about 0.45 kb) containing the most part of the TNF structural gene. On the other hand, pIN5T8 was completely decomposed by use of SalI and EcoRI to obtain a DNA fragment (about 2.8 kb) containing lpp promoter, a replication initiation site and a portion of the tetracycline resistant gene. Also, pIN5T8 was cleaved by SalI alone to yield a DNA fragment (about 0.7 kb) containing the TrpA terminator.

The three types of DNA fragments obtained above and a chemically synthesized EcoRI-AvaI linker DNA fragment
$\left[\begin{array}{l}\text{5'-AATTCATGTCTCGAACC} \\ \qquad \text{GTACAGAGCTTGGGGCT-5'}\end{array}\right]$ were mixed and joined with use of T4DNA ligase to construct pIN5$^{\Delta N4}$TNFST8. Then, pIN5$^{\Delta N4}$TNFST8 was digested by HindIII and NdeI to separate a DNA fragment (about 1.9 kb) containing the lpp promoter, the $^{\Delta N4}$TNF structural gene and the TrpA terminator. On the

other hand, pBR322ΔBalI (refer to Fig. 10) was decomposed by HindIII and NdeI to separate a DNA fragment (about 1.6 kb) containing the tetracycline-resistant gene. The two types of DNA fragments were mixed and joined with T4DNA ligase to pIN5$^{\Delta N4}$TNFST8$^{rop-}$.

2. Production of $^{\Delta N4}$TNF by use of transformed bacteria

Plasmids for producting $^{\Delta N4}$TNF obtained in Item 1 were introduced into E. coli W3110 and WA802, respectively to yield transformant showing tetracycline resistance. As to pPLT4$^{\Delta N4}$TNFST8, W3110/CI or WA802/CI which had been transformed with plasmid pCI857 which is capable of expressing a temperature-sensitive repressor gene (CI857) of λ phage, is compatible with pBR322 and is capable of being screened by a kanamycin-resistant gene as a marker, was used as the host. Examples of $^{\Delta N4}$TNF-producing transformant are shown below.

a) Production of $^{\Delta N4}$TNF by culture in a flask

Transformant WA802/pT4$^{\Delta N4}$TNFST8$^{rop-}$ and W3110/pT4$^{\Delta N4}$TNFST8$^{rop-}$ each were inoculated in an LB medium (1% trypton, 0.5% yeast extract, 0.5% NaCl, pH 7.0) containing 10 μg/ml of tetracycline, and were cultured at 37$^{o}$C until the latter stage of logarithmic phase of bacterial growth. Two milliliter portion of each of the culture mixture was added to 200 ml of a GC medium (2% glycerin, 3% casamino acid, 0.5% $K_2HPO_4$, 0.2% yeast extract, 0.1% $MgSO_4$ $7H_2O$, pH 6.5) containing 10 μg/ml of tetracycline, and the mixture was cultured in a 1,000 ml Erlenmeyer flask with shaking at 250 rpm at 37$^{o}$C for 15 hours. The amount of bacterial cells and the TNF productivity were measured for the culture mixture each. The results are shown in Table 3. OD660 indicates the amount of bacterial cells, and the TNF activities are shown in terms of activities of samples prepared according to the following method. That is, a 0.5 ml portion of the culture mixture was subjected to centrifugation, and the bacterial cells obtained were lysed by suspending them with 0.5 ml of 250 μg/ml of lysozyme (Grade 1: Egg alubmin lysozyme manufactured by Sigma Co.) in a PBS solution (manufactured by Nissui Seiyaku Co., Ltd.),

reacting the suspension at 0°C for 30 minutes, and repeating three times rapid freezing on an ethanol-dry ice bath and thawing at 37°C. The lysed mixture was subjected to centrifugation at 12,000 rpm for 5 minutes, and the supernatant was used as a sample for measurement of TNF activities.

Also, analysis of the bacterial cells were carried out according to SDS polyacrylamide gel electrophoresis (SDS PAGE) as follows. That is, a 0.2 ml portion of the culture mixture was subjected to centrifugation to collect the bacterial cells, and the cells obtained by removal of the supernatant were suspended in 0.15 ml of a SDS sample solution (10% glycerin, 5% 2-mercapto-ethanol, 2.3% SDS, 62.5 mM tris hydrochloric acid, pH 6.8). The suspension was heated at 100°C for 10 minutes. A portion of the mixture was subjected to electrophoretis separation by 15% SDS PAGE according to a conventional method before protein dyeing with Coomassie Blue. As a result, a band corresponding to $^{\Delta N4}$TNF protein having a molecular weight of about 17 K was detected.

In contrast, the host E. coli which has not been transformed and cultured in a manner similar to that described above showed no band corresponding to the protein of 17 K.

Table 3: Production of $^{\Delta N4}$TNF by culture in a flask

| Transformed strain | OD660 | TNF activities (unit/ml) |
|---|---|---|
| W3110/pT4$^{\Delta N4}$TNFST8$^{\text{rop}-}$ | 6.6 | $1.7 \times 10^6$ |
| WA802/pt4$^{\Delta N4}$TNFST8$^{\text{rop}-}$ | 5.2 | $1.0 \times 10^6$ |

b) production of $^{\Delta N4}$TNF by use of a jar fermenter E. coli WA802/CI/pPLT4$^{\Delta N4}$TNFST8, WA802/pT4$^{\Delta N4}$TNFST8$^{\text{rop}-}$ and W3110/pT4$^{\Delta N4}$TNFST8$^{\text{rop}-}$ each, which were transformed by plasmids pPLT4$^{\Delta N4}$TNFST8 and pT4$^{\Delta N4}$TNFST8$^{\text{rop}-}$ obtained in Items 1-a) and 1-b) above, were cultivated on 2 ℓ of the GC medium described in Item 2-a) by use of a jar fermenter. As seed bacteria, a culture mixture obtained by cultivating the above bacteria in 20 ml of an LB medium containing 10 µg/ml of tetracycline until the latter logarithmic phase of bacterial growth was used, and the culture was carried out at $37^{O}$C for 13 hours by maintaining the culture liquids at pH 7.0 with a 5N NaOH and also controlling dissolved oxygen at 1 ppm - 2.5 ppm. After culture a portion of the culture mixture was sampled, and a crude extract solution was prepared according to the method described in Item 2-a) to investigate the TNF activities. The results are shown in Table 4.

As seen from the table, each of the transformed bacteria showed high TNF activities. The fact that the same transformed bacteria show the productivities higher than those in Table 3 is probably due to a difference in culture conditions. Also, the culture liquids of the above transformed bacteria were subjected to SDS PAGE according to the method described above [Item 2-a)]. As a results, a thick protein band was noted at a position of about 17 K molecular weight corresponding to $^{\Delta N4}$TNF polypeptide, from which it was recognized that the transformed bacteria produce $^{\Delta N4}$TNF in high yields.

Table 4: Production of $^{\Delta N4}$TNF by means of a jar fermenter

| Transformed bacteria | OD660 | TNF activities (unit/ml) |
|---|---|---|
| WA802/CI/pPLT4$^{\Delta N4}$TNFST8 | 22.1 | $5.7 \times 10^{6}$ |
| WA802/pT4$^{\Delta N4}$TNFST8$^{rop-}$ | 18.7 | $2.9 \times 10^{7}$ |
| W3110/pT4$^{\Delta N4}$TNFST8$^{rop-}$ | 22.7 | $3.0 \times 10^{7}$ |

3. Measurements of TNF activities

Measurements of TNF activities were carried out as follows according to a slight improvement of the method described in Ruff, M. R. and Gifford, E. G. Lymphokines, (Ed. Pick, E.) vol 2, 235 - 272, Academic Press, New York (1981). That is, 0.1 ml of a $6 \times 10^{5}$ cells/ml suspension (in the medium below) of L-929 cell (ATCC entrusted No. CCL-1) which was a substrain of a mouce L-cell and was in the logarithmic phase of bacterial growth was added to each hole of a flat bottom type tissue culture microplate with 96 holes (manufactured by Nunc Co.) each of which contains 0.1 ml of a sample diluted stepwise with an Eagle MEM medium containing 10% fetal bovine serum, 0.1% $NaHCO_3$ and 0.03% glutamine. Actinomycin D (manufactured by Makor Chemical Co.) was added beforehand to each hole so that it amounts finally to 1 µg/ml. Then, the microplate described above was treated at 37°C for about 18 hours in air containing 5% $CO_2$ for culture. After culture, the supernatant was removed, and 0.1 ml of an aqueous 0.5% Crystal Violet solution containing 20% methanol was added to each hole so as to stain the L-929 cells adhering thereto for 15 minutes at room temperature. The stained microplate was washed with water, and 0.1 ml of a 33% acetic acid aqueous solution was added to extract pigments from the stained cells. The absorbance of the extract at 577 nm was measured by a Titertek Multiskan Spectrophotometer. The reciprocal of a dilution magnification of the sample showing an absorbance corresponding to 50% of the absorbance of the control was used to represent the TNF activities of the sample in terms of unit/ml. At least 4 runs of measurements were carried out for one sample, and the average value was calculated.

4. Purification of polypeptide $^{\Delta N4}$TNF

The method of purification of polypeptide having TNF activities was described in Example 1 above. This Example 2 describes a process for purifying the polypeptide having TNF activities from cultured bacterial body of WA802/pT4$^{\Delta N4}$TNFST8$^{rop-}$, E. coli transformed by plasmid pT4$^{\Delta N4}$TNFST8$^{rop-}$ obtained in Item 1 above.

Recombinant E. coli WA802/pT4$^{\Delta N4}$TNFST8$^{rop-}$ was cultivated with shaking in 2 ℓ of a GC medium (2% glycerin, 3% casamino acid, 0.5% $KH_2PO_4$, 0.2% yeast extract, 0.1% $MgSO_4 \cdot 7H_2O$, pH 6.5) containing tetracycline (10 µg/ml) at 37°C for 14 hours by use of a 3 ℓ jar fermenter. After collection of bacterial cells by centrifugation, the cells were suspended in a 10 mM tris-hydrochloric acid buffer (pH 8.0) (will be abbreviated as Tris-buffer (pH 8.0) hereinafter) amounting to about 7 times as much as the amount of the wet bacterial cells. The cells were disrupted by use of a Manton-Gaulin Laboratory Homogenizer 15M-8TA with cooling. The mixture obtained was subjected to centrifugation at 8,000 rpm for 20 minutes. Then, poly-ethyleneimine (PEI: manufactured by BASF Co.) was added to a concentration of 0.2% to a 700 ml portion of the supernatant, and the mixture was centrifuged at 8,000 rpm for 30 minutes to yield 680 ml of a supernatant fraction (crude extract). The crude extract had TNF activities of $1.25 \times 10^7$ unit/ml ($7.8 \times 10^6$ unit/mg protein). Then, ammonium sulfate was added to the crude extract to an 80% saturation, and the mixture was centrifuged to precipitate proteins. The proteins obtained were dissolved in 120 ml of a 10 mM Tris-buffer (pH 8.0), and, after sufficiently dialyzed against the same buffer, were subjected to anion exchange chromatography with a carrier 'DEAE Toyopearl 650 C' (manufactured by Toyo Soda Co.) sufficiently equilibrated with the buffer. After sufficiently washing the column with the Tris-buffer (pH 8.0), NaCl solution in the Tris-buffer (pH 8.0) having a linear concentration gradient from 0 mM to 300 mM was used to elute the adsorbed proteins. Fractions having TNF activities were eluted at a NaCl concentration of about 50 mM to 60 mM (Fig. 12). Then, a 100 ml portion of the fractions having TNF activities was diluted with a Tris-buffer (pH 7) to make up the total volume to 200 ml, and the solution was subjected to column chromatography using DEAE Sepharose CL-6B (manufactured by Pharmacia Co.) equilibrated with the Tris-buffer (pH 7). After sufficiently washing with the buffer, a NaCl solution in the Tris-buffer (pH 7) having a linear concentration gradient from 0 mM to 200 mM was used for elution. As a result, a large porption of the proteins were eluted at a NaCl concentration of 60 mM to 70 mM (Fig. 13), and fractions having TNF activities were also eluted in the same concentration. Then, a 120 ml portion of the fractions showing TNF activities was subjected to column chromatography on Matrex Blue A (manufactured by Amicon Co.), a dye adsorption affinity carrier, sufficiently equilibrated with the Tris-buffer (pH 7). Fractions having TNF activitires or protein were eluted with the Tris-buffer (pH 8). Also, the peak of the protein eluted was in good conformity with the peak of TNF activities (Fig. 14).

This indicates that the protein having TNF activities is specifically adsorbed by Matrex Blue A at pH 7.0 and is desorbed at pH 8.0. In contrast, the polypeptide consisting of 157 amino acids shown in Fig. 1 is adsorbed by the above carrier at both of pH 7.0 and pH 8.0. Therefore, it is necessary to increase the salt concentration, for example, by use of sodium chloride, in the Tris-buffer (pH 8) for the desorption of the polypeptide from the carrier.

The polypeptide purified by column chromatography on the above dye adsorption affinity carrier showed an approximately single band by SDS PAGE. This indicates that the polypeptide has a purity of 95% or more at the stage. The polypeptide having TNF activities obtained above was adsorbed again on DEAE Sepharose CL-6B equilibrated with the Tris-buffer (pH 7), and was eluted by a solution having a constant pH of 7.0 and having simultaneous linear gradients of the tris-hydrochloric acid concentration and the NaCl concentration of from 10 mM to 200 mM and 0 mM to 100 mM, respectively to purify the polypeptide at a purity of 99% or more. The polypeptide was used as the final $^{\Delta N4}$TNF sample. The authentic sample had TNF activities of $2.2 \times 10^7$ unit/mg of protein on the average, and it could be further purified about 13 times in an about 60% yield from the crude extract. The authentic sample could further be purified by use of Sephacryl S-200, a gel filtration carrier. The results of purification at each process are shown in Table 5. Also, the SDS PAGE pattern of the fraction having TNF activities from Sephacryl S-200 column chromatography was shown in Fig. 21.

## Table 5
### Results of purification at each process

| Process purification | Volume (ml) | Protein (mg/ml) | Total protein (mg) | Total TNF activities (unit) | Specific activities (unit/mg protein) | Recovery (%) |
|---|---|---|---|---|---|---|
| Supernatant cell homogenate | 700 | 10.6 | 7,420 | $1.26 \times 10^{10}$ | $1.7 \times 10^6$ | 100 |
| Treatment with 0.2% PEI (Crude extract) | 680 | 1.6 | 1,088 | $0.85 \times 10^{10}$ | $7.8 \times 10^6$ | 67 |
| Precipitation with 80% saturated ammonium sulfate | 175 | 5.7 | 997 | $1.09 \times 10^{10}$ | $1.09 \times 10^6$ | 87 |
| DEAE-Toyopearl (pH 8.0) | 100 | 4.5 | 450 | $0.63 \times 10^{10}$ | $13.8 \times 10^6$ | 50 |
| DEAE Sepharose CL-6B (pH 7.0) | 120 | 3.0 | 360 | $0.75 \times 10^{10}$ | $20.8 \times 10^6$ | 60 |
| Matrex Blue A | 117 | 2.9 | 340 | $0.73 \times 10^{10}$ | $21.5 \times 10^6$ | 58 |
| DEAE Sepharose CL-6B (pH 7.0) | 100 | 2.8 | 336 | $0.75 \times 10^{10}$ | $22.3 \times 10^6$ | 60 |

5.  Physical and chemical properties of the polypeptide
    purified

a)  Amino acid composition (refer to Table 6)

The fraction having TNF activities obtained by DEAE Sepharose CL-6B coumn chromatography after Matrex Blue A column chromatography in the above item was further purified by a reverse-phase high performance liquid chromatography (Shimadzu Seisakusho Ltd.) using a Microbonder pack $C_{18}$ column (manufactured by Waters Co.) before amino acid analysis. About 15 µg of a sample was hydrolyzed with 100 µl of 6N HCl in a sealed tube under reduced pressure at $110^{\circ}C$ for 24 hours and 72 hours, and the hydrolyzed product was dried under reduced pressure. Then, 200 µl of 0.02N HCl was added to the product to determine the amino acid composition by means of an automatic amino acid analyzer (Model 835-50 manufactured by Hitachi Seisakusho Ltd). The ratio of the number of molecules for each amino acid are shown in Table 6 with the number of molecules of leucine (Leu) as standard (18.0). The results were close to the theoretical amino acid composition of $^{\Delta N4}$TNF. In the table, the value for tryptophan (Trp) is a value determined after hydrolysis (in a sealed tube at $110^{\circ}C$ for 24 hours) with 3 moles of mercaptoethane sulfonic acid, and the value for cysteine (Cys) is value determined for cysteinic acid after oxidation with performic acid and hydrolysis (at $110^{\circ}C$ for 24 hours) with 6N HCl.

Concerning the presence of methionine (Met) at the amino terminus in the purified polypeptide, the above amino acid analysis revealed that the purified polypeptide has about 5% formylated or free form of methionine.

Table 6: Analytical results of amino acid composition

| Animo acid | Theoretical value | Observed value |
|:---:|:---:|:---:|
| Trp | 2 | 2.2 ± 0.2 |
| Asp | 12 | 12.1 ± 0.5 |
| Thr | 6 | 6.1 ± 0.3 |
| Ser | 11 | 11.4 ± 0.4 |
| Glu | 20 | 19.9 ± 0.8 |
| Pro | 10 | 9.6 ± 0.4 |
| Gly | 11 | 11.1 ± 0.4 |
| Ala | 13 | 13.4 ± 0.5 |
| Cys | 2 | 1.6 ± 0.2 |
| Val | 12 | 11.7 ± 0.5 |
| Met | 0 | (0.05) |
| Ile | 8 | 7.5 ± 0.4 |
| Leu* | 18 | 18.0 |
| Tyr | 7 | 6.8 ± 0.3 |
| Phe | 4 | 4.0 ± 0.2 |
| Lys | 6 | 6.0 ± 0.3 |
| His | 3 | 3.1 ± 0.2 |
| Arg | 8 | 8.0 ± 0.4 |

* The number of molecules of leucine was adopted as standard (18.0), and the number of molecules of each animo acid was represented by a ratio to the standard.

b) Comparison of TNF activities

The specific $^{\Delta N4}$TNF activity of the final authentic sample obtained in Item 4 in the Example was shown in Table 5. The inventors further investigated the TNF activity (specific activity) of a polypeptide (TNF) which was purified in a manner similar to that in Item 4 in the Example from cultured bacterial cells of E. coli WA802/pT4TNFST8$^{rop-}$ that has an ability to produce a polypeptide consisting of 157 amino acids (Fig. 1: TNF) and was transformed by plasmid pT4TNFST8$^{rop-}$ obtained by improvement of plamsid pBR322-PL-T4-hTNF (DSM 3175). The results are shown in Table 7. The specific activity of $^{\Delta N4}$TNF was 5.9 - 10 x $10^7$ unit/mg protein, whereas that of the polypeptide consisiting of 157 amino acids was 2.6 - 6.2 x $10^7$ unit/mg protein. Thus, it was noted that $^{N4}$TNF has a specific activity higher than that of the polypeptide consisting of 157 amino acids. Also, a slight difference in the TNF activities of $^{N4}$TNF in Table 5 and Table 7 will probably be due to errors in measurements.

The results of SDS PAGE of both polypeptides purified are shown in Fig. 21. Both showed obviously different patterns. The estimated molecular weight of $^{N4}$TNF was about 17,000 $\pm$ 1,500 in comparison with the electrophoretic patterns of standard proteins.

Table 7: Comparison of bactercidal activities between $^{\Delta N4}$TNF produced by recombinant E. coli and the polypeptide (TNF) consisting of 157 amino acids

| Experiments | | Activity (unit/ml) | Protein (mg/ml) | Specific activity (unit/mg protein) |
|---|---|---|---|---|
| I | $^{\Delta N4}$TNF | 3.0 x $10^7$ (n=4) | 0.39 | 10.0 x $10^7$ |
| | TNF | 1.8 x $10^7$ (n=4) | 0.29 | 6.2 x $10^7$ |
| | | 1.7 x $10^7$ (n=4) | 0.31 | 5.5 x $10^7$ |
| II | $^{\Delta N4}$TNF | 1.6 x $10^7$ (n=4) | 0.27 | 5.9 x $10^7$ |
| | TNF | 3.9 x $10^7$ (n=4) | 1.49 | 2.6 x $10^7$ |
| | | 5.2 x $10^7$ (n=4) | 1.68 | 3.1 x $10^7$ |

c) Isoelectric point (refer to Fig. 22)

The isoelectric pionts were measured by chromato-focusing for $^{\Delta N4}$TNF and the polypeptide consisting of 157 amino acids (Fig. 1). Before the chromatofocusing, both of the polypeptides were purified by a due adsorption affinity chromatography followed by DEAE-Sepharose CL-6B chromatography (Item 4 in the Example: refer to Fig. 20).

Chromatofocusing was carried out by use of PBE 94 (manufactured by Pharmacia Co.), a column for chromatofocusing use, and flowing a 10-fold dilution of Polybuffer-74 (manufactured by Pharmacia Co.) adjusted at pH 4.0 with hydrochloric acid through the column to make a pH gradient to elute both polypeptides. The results are shown in Fig. 22. $\Delta N4$TNF was eluted at a pH of 5.4 - 5.1 (5.2 ± 0.2), whereas the polypeptide consisting of 157 amino acids were eluted at a pH of 5.7 - 5.3 (5.5 ± 0.2). Thus, it was noted that the isoelectric points of the two polypeptides are oviously different by chromatofocusing measurements.

d) thermal and pH stabilities (refer to Fig. 15-b and Fig. 16-b)

The pH and the thermal stabilities were investigated for fractions containing $\Delta N4$TNF and the polypeptide consisting of 157 amino acids (refer to Fig. 1). Both polypeptides were purified by a dye adsorption affinity chromatography followed by DEAE Sepharose chromatography (Item 4 in the Example: Fig. 20). As to the pH stability, the above samples having the same stabilities were dialyzed against a sufficient amount of buffer adjusted at each pH shown in Fig. 16-b at 4°C for 14 hours, and then against a 10 mM tris-hydrochloric acid buffer (pH 7.5) at 4°C for 1 hour to measure the residual activities. The results are shown in Fig. 16-b. As can be seen from the results, the residual activity of $\Delta N4$TNF was higher than that of the polypeptide consisting of 157 amino acids at a pH of 11 or more and 4.5 or less.

As to the thermostability, the residual activities were observed for the above samples after causing to stand for 30 minutes at each temperature shown in Fig. 15-b. As can be seen from the results, $\Delta N4$TNF showed residual activities higher than those of the polypeptide consisting of 157 amino acids at a temperature of 75°C.

Thus, it was revealed that $N4$TNF is a polypeptide obviously different from and superior to the conventionally known polypeptide consisting of 157 amino acids in terms of physical and chemical properties.

6.  $^{\Delta N4}$TNF preparations

One mg of $^{\Delta N4}$TNF of a 99% or more purity obtained in Item 4 above, 50 mg of mannitol and 9 mg of sodium chloride were dissolved in 1 ml of a phosphoric acid buffer (pH 7.2), and the solution was freeze-dried to prepared a $^{\Delta N4}$TNF preparation.  The preparation was dissolved in 1 ml of pure water, and the resulting solution was injected intravenously or at the affected part in tumor-bearing mice at a dose of 0.2 ml to 1 ml per kg.

Also, a preparation containing 1 mg of $^{\Delta N4}$TNF, 50 mg of mannitol, 9 mg of sodium chloride and 1 mg each of serum albumin or gelatin, and a preparation containing 1 mg of $^{\Delta N4}$TNF, 50 mg of mannitol, 9 mg of sodium chloride, 10 mg of glycine or 0.1 mg of polyethylene glycol 4000 were prepared. Each preparation was formed by freeze-drying after dissolution in 1 ml of a phosphoric acid buffer of pH 7.2.

The $^{\Delta N4}$TNF preparations should not be limited to the above compositions, and the pH of the phosphoric acid buffer may be 5.5 - 9.0, and 1 ml of the phosphoric acid buffer may contain 0.05 - 5.0 mg of $^{\Delta N4}$TNF and 8 - 12 mg of sodium chloride.

Reference Example

A culture of E. coli transformant, WA802/pT4TNFST8$^{rop-}$, constructed by the recombinant DNA technology so as to produce TNF active polypeptide made of 157 amino acids (see Fig. 1) was subjected to the same purification steps as shown above, and the polypeptide obtained had TNF activity which was at least 99% pure.

Since the purification steps used to obtain this polypeptide were the same as those described above, details thereof are replaced by Figs. 17 to 20 which show the chromatograms obtained in the respective steps.

Fig. 17 shows the result of column chromatography on DEAE Sepharose CL-6B performed in the second step of the purification scheme; Fig. 18 shows the result of column chromatography on Matrex Blue A performed in the third step; Fig. 19 shows the result of subsequent chromatography on

0205038

Phenyl-Sepharose CL-4B; and Fig. 20 shows the result of chromatography on Sephacryl S-200.

Two samples of polypeptide having TNF activity were obtained by the above procedures of purification; as shown in Fig. 21, SDS PAGE analysis of these samples revealed that they were substantially pure and made of polypeptides with apparently different sizes.

The method of purification from the culture of recombinant microorganism described above could also be applied to the purpose of purification from cultures of other recombinant E. coli strains having the ability to produce polypeptide with TNF activity, as well as to the purification from recombinant E. coli cells cultured on media other than those used in Example 2.

Claims:

1. A novel polypeptide having antitumor activity wherein the first 23 $NH_2$-terminal amino acid sequence of its monomeric form is

Ser - Arg - Thr - Pro - Ser - Asp - Lys - Pro - Val -
Ala - His - Val - Val - Ala - Asn - Pro - Gln - Ala -
Glu - Gly - Gln - Leu - Gln

and which has the following characteristics:

(a) a molecular weight of 17,000 ± 1,500 as calculated from data obtained by SDS polyacrylamide electrophoresis;

(b) an isoelectric point of 5.2 ± 0.2 as determined by chromatofocusing;

(c) a cytocidal activity against L-929 cells of $1 \times 10^7$ - $10 \times 10^7$ units/mg protein as defined hereinafter; and

(d) ability to adsorb on a dye adsorption affinity carrier.

2. A polypeptide according to Claim 1 wherein a molecular weight of 47,000 ± 5,000 as calculated from data obtained by gel permeation chromatography, an isoelectric point of 5.2 - 5.3 as determined by chromatofocusing, and a cytocidal activity against L-929 cells of $1 \times 10^7$ - $10 \times 10^7$ units/mg protein.

3. A polypeptide according to Claim 1 which is obtained from a culture broth of activated human macrophage-like cells.

4. A polypeptide according to Claim 3 wherein the human macrophage-like cells derived from histiocytic lymphoma.

5. A polypeptide according to Claim 4 wherein the cells derived from histiocytic lymphoma are U-937 (American Type Culture Collection, CRL 1593).

6. A polypeptide according to Claim 3 wherein the human macrophage-like cells are activated by addition of 4β-phorbol 12β-myristage 13α-acetate (PMA), retinoic acid and insulin to the culture broth.

7. A substantially pure polypeptide having antitumor activities which is obtained by culturing microorganism transformed by a plasmid which contains a DNA fragment having a promoter region and a ribosome binding (or SD) sequence upstream a DNA sequence coding for the following amino acid sequence so as to express said DNA sequence, isolating and purifying the polypeptide from the culture: said amino acid sequence being Ser-Arg-Thr-Pro-Ser-Asp-Lys-Pro-Val-Ala-His-Val-Val-

20
Ala-Asn-Pro-Gln-Ala-Glu-Gly-Gln-Leu-Gln-Trp-Leu-Asn-

Arg-Arg-Ala-Asn-Ala-Leu-Leu-Ala-Asn-Gly-Val-Glu-Leu-

40
Arg-Asp-Asn-Gln-Leu-Val-Val-Pro-Ser-Glu-Gly-Leu-Tyr-

60
Leu-Ile-Tyr-Ser-Gln-Val-Leu-Phe-Lys-Gly-Gln-Gly-Cys-

Pro-Ser-Thr-His-Val-Leu-Leu-Thr-His-Thr-Ile-Ser-Arg-

80
Ile-Ala-Val-Ser-Tyr-Gln-Thr-Lys-Val-Asn-Leu-Leu-Ser-

100
Ala-Ile-Lys-Ser-Pro-Cys-Gln-Arg-Glu-Thr-Pro-Glu-Gly-

Ala-Glu-Ala-Lys-Pro-Trp-Tyr-Glu-Pro-Ile-Tyr-Leu-Gly-

120
Gly-Val-Phe-Gln-Leu-Glu-Lys-Gly-Asp-Arg-Leu-Ser-Ala-

140
Glu-Ile-Asn-Arg-Pro-Asp-Tyr-Leu-Asp-Phe-Ala-Glu-Ser-

153
Gly-Gln-Val-Tyr-Phe-Gly-Ile-Ile-Ala-Leu.

8.    A polypeptide according to Claim 7 wherein the polypeptide has a specifc TNF (Tumor Necrosis Factor which will be defined in the specification) activity of 1 x $10^7$ to 10 x $10^7$ unit/mg protein.

9.    A polypeptide according to Claim 7 wherein the polypeptide has an amino acid composition as shown in Table 6 in the specification.

10.    A polypeptide according to Claim 7 wherein the polypeptide is absorbed at pH 7.0 on, and desorbed at pH 8.0 from a color adsorption affinity carrier.

11.    A polypeptide according to Claim 7 wherein the amino acid sequence is represented by

X-Ser-Arg-Thr-Pro-Ser-Asp-Lys-Pro-Val-Ala-His-Val-

20
Val-Ala-Asn-Pro-Gln-Ala-Glu-Gly-Gln-Leu-Gln-Trp-Leu-

Asn-Arg-Arg-Ala-Asn-Ala-Leu-Leu-Ala-Asn-Gly-Val-Glu-

40
Leu-Arg-Asp-Asn-Gln-Leu-Val-Val-Pro-Ser-Glu-Gly-Leu-

60
Tyr-Leu-Ile-Tyr-Ser-Gln-Val-Leu-Phe-Lys-Gly-Gln-Gly-

Cys-Pro-Ser-Thr-His-Val-Leu-Leu-Thr-His-Thr-Ile-Ser-

80
Arg-Ile-Ala-Val-Ser-Tyr-Gln-Thr-Lys-Val-Asn-Leu-Leu-

100
Ser-Ala-Ile-Lys-Ser-Pro-Cys-Gln-Arg-Glu-Thr-Pro-Glu-

Gly-Ala-Glu-Ala-Lys-Pro-Trp-Tyr-Glu-Pro-Ile-Tyr-Leu-

120
Gly-Gly-Val-Phe-Gln-Leu-Glu-Lys-Gly-Asp-Arg-Leu-Ser-

140
Ala-Glu-Ile-Asn-Arg-Pro-Asp-Tyr-Leu-Asp-Phe-Ala-Glu-

153
Ser-Gly-Gln-Val-Tyr-Phe-Gly-Ile-Ile-Ala-Leu.

wherein X represents a hydrogen atom or a methionine residue.

12.    A method of purifying polypeptide defined in Claim 2 and having antitumor activity or tumor necrotic effect, wherein a dye adsorption affinity carrier is used in the step of purifying said polypeptide from a culture broth containing the same.

13.    A method according to Claim 12 wherein any two of the following chromatographic carriers are used in combination: (a) an anion exchanging carrier; (b) a hydrophobic carrier; (c) a chromatofocusing carrier and (d) a gel permeation carrier.

14.    A method according to Claim 12 wherein the culture broth containing the polypeptide having antitumor activity or tumor nectrotic effect is obtained by culturing human macrophage-like cells.

15.    A method according to Claim 12 wherein the culture broth containing the polypeptide having antitumor activity or tumor necrotic effect is obtained by culturing a microorganism or cells constructed by the recombinant DNA technology.

16.    A process for preparing a novel polypeptide having antitumor activities which comprises culturing a microorganism transformed by a plasmid comprising a DNA fragment having a promoter region and a ribosome binding (or SD) sequence upstream a DNA sequence coding for the following amino acid sequence so as to express said DNA sequence, extracting a crude polypeptide from the culture, and purifying the crude polypeptide:   said amino acid sequence being Ser-Arg-Thr-Pro-Ser-Asp-Lys-Pro-Val-Ala-His-Val-Val-

20
Ala-Asn-Pro-Gln-Ala-Glu-Gly-Gln-Leu-Gln-Trp-Leu-Asn-

Arg-Arg-Ala-Asn-Ala-Leu-Leu-Ala-Asn-Gly-Val-Glu-Leu-

40
Arg-Asp-Asn-Gln-Leu-Val-Val-Pro-Ser-Glu-Gly-Leu-Tyr-

60
Leu-Ile-Tyr-Ser-Gln-Val-Leu-Phe-Lys-Gly-Gln-Gly-Cys-

Pro-Ser-Thr-His-Val-Leu-Leu-Thr-His-Thr-Ile-Ser-Arg-

80
Ile-Ala-Val-Ser-Tyr-Gln-Thr-Lys-Val-Asn-Leu-Leu-Ser-

100
Ala-Ile-Lys-Ser-Pro-Cys-Gln-Arg-Glu-Thr-Pro-Glu-Gly-

Ala-Glu-Ala-Lys-Pro-Trp-Tyr-Glu-Pro-Ile-Tyr-Leu-Gly-

120
Gly-Val-Phe-Gln-Leu-Glu-Lys-Gly-Asp-Arg-Leu-Ser-Ala-

140
Glu-Ile-Asn-Arg-Pro-Asp-Tyr-Leu-Asp-Phe-Ala-Glu-Ser-

153
Gly-Gln-Val-Tyr-Phe-Gly-Ile-Ile-Ala-Leu.

17.    A process according to Claim 16 wherein the DNA sequence coding for said amino acid sequence is a sequence represented by sequence (I) or a sequence haivng biological functions substantially equivalent thereto:

```
          10        20        30        40        50
TCTCGAACCCCGAGTGACAAGCCTGTAGCCCATGTTGTAGCAAACCCTCAAG

          60        70        80        90        100
CTGAGGGGCAGCTCCAGTGGCTGAACCGCCGGGCCAATGCCCTCCTGGCCAA

          110       120       130       140       150
TGGCGTGGAGCTGAGAGATAACCAGCTGGTGGTGCCATCAGAGGGCCTGTAC

          160       170       180       190       200
CTCATCTACTCCCAGGTCCTCTTCAAGGGCCAAGGCTGCCCCTCCACCCATG

          210       220       230       240       250       260
TGCTCCTCACCCACACCATCAGCCGCATCGCCGTCTCCTACCAGACCAAGGT

          270       280       290       300       310
CAACCTCCTCTCTGCCATCAAGAGCCCCCTGCCAGAGGGAGACCCCAGAGGGG

          320       330       340       350       360
GCTGAGGCCAAGCCCTGGTATGAGCCCATCTATCTGGGAGGGGTCTTCCAGC

          370       380       390       400       410
TGGAGAAGGGTGACCGACTCAGCGCTGAGATCAATCGGCCCGACTATCTCGA

          420       430       440       450       459
CTTTGCCGAGTCTGGGCAGGTCTACTTTGGGATCATTGCCCTG
```

18.    A process according to Claim 17 wherein the DNA sequence coding for said amino acid sequence is provided with a triplet coding for translation initiation at the upstream terminal region of sequence (I) and a triplet coding for translation termination at the downstream terminal region thereof.

19.    A process according to Claim 18 wherein a transcription terminator coding for termination of transcription which is derived from E. coli is joined just downstream the triplet coding for translation terminaiton.

20.    A process according to Claim 19 wherein the transcription terminator is a TrpA terminator.

21.    A process according to Claim 19 or 20 wherein the DNA sequence which has the transcription terminator derived from E. coli and is joined just downstream the triplet coding for termination of translation is represented by the following sequence:

ATGTCTCGAACCCCGAGTGACAAGCCTGTAGCCCATG
TTGTAGCAAACCCTCAAGCTGAGGGGCAGCTCCAGTG
GCTGAACCGCCGGGCCAATGCCCTCCTGGCCAATGGC
GTGGAGCTGAGAGATAACCAGCTGGTGGTGCCATCAG
AGGGCCTGTACCTCATCTACTCCCAGGTCCTCTTCAA
GGGCCAAGGCTGCCCCTCCACCCATGTGCTCCTCACC
CACACCATCAGCCGCATCGCCGTCTCCTACCAGACCA
AGGTCAACCTCCTCTCTGCCATCAAGAGCCCCTGCCA
GAGGGAGACCCCAGAGGGGGCTGAGGCCAAGCCCTGG
TATGAGCCCATCTATCTGGGAGGGGTCTTCCAGCTGG
AGAAGGGTGACCGACTCAGCGCTGAGATCAATCGGCC
CGACTATCTCGACTTTGCCGAGTCTGGGCAGGTCTAC
TTTGGGATCATTGCCCTGTGAG TCGACAGCCCGCCT
S

AATGAGCGGGCTTTTTTTTCTCGG AATTCTCATGT
E

TTGACAGCTTATCATCGATAAGCTTTAATGCGGTAG

wherein ATG represents the triplet coding for translation initiation, TGA represents the triplet coding for translation termination, ↔ represents a TrpA terminatror, $\overset{\downarrow}{S}$ represents the cutting site with SalI and $\overset{\downarrow}{E}$ represents the cutting site with EcoRI.

22. A process according to Claim 16 wherein the promoter region comprises a $P_L$ promoter originating from $\lambda$ phage or a $T_4$ phage promoter or both of them.

23. A process according to Claim 16 wherein the promoter region is derived from a gene coding for klipoprotein of the outer cell membrane of E. coli.

24. A process according to Claim 16 wherein the ribosome binding (or SD) sequence is a DNA sequence derived from $T_4$ phage or a chemically synthesized DNA sequence.

25. A process according to Claim 16 wherein the plasmid is E. coli plasmid pBR322 from which the rop (repressor of primer) DNA region controlling replication of the plastic DNA is removed.

26. A process according to Claim 16 wherein the plasmid is $pPLT4^{\Delta N4}TNFST8$, $pT4^{\Delta N4}TNFST8^{rop-}$ or $pIN5^{\Delta N4}TNFST8^{rop-}$.

27.    A process according to Claim 16 wherein the microorganism is *E. coli*.

28.    *Escherichia coli* transformed by a plasmid represented by $pPLT4^{\Delta N4}TNFST8$, $pT4^{\Delta N4}TNFST8^{rop-}$ or $pIN5^{\Delta N4}TNFST8^{rop-}$.

29.    *Escherichia coli* according to Claim 28 which is represented by $WA802/CI/pPLT4^{\Delta N4}TNFST8$, $W3110/CI/pPLT4^{\Delta N4}TNFST8$, $WA802/pT4^{\Delta N4}TNFST8^{rop-}$, $W3110/pT4^{\Delta N4}TNFST8^{rop-}$, $WA802/pIN5^{\Delta N4}TNFST8^{rop-}$ or $W3110/pIN5^{\Delta N4}TNFST8^{rop-}$.

30.    A pharmaceutical composition comprising as an active ingredient, a polypeptide having the following amino acid sequence:

X-Ser-Arg-Thr-Pro-Ser-Asp-Lys-Pro-Val-Ala-His-Val-

20
Val-Ala-Asn-Pro-Gln-Ala-Glu-Gly-Gln-Leu-Gln-Trp-Leu-

Asn-Arg-Arg-Ala-Asn-Ala-Leu-Leu-Ala-Asn-Gly-Val-Glu-

40
Leu-Arg-Asp-Asn-Gln-Leu-Val-Val-Pro-Ser-Glu-Gly-Leu-

60
Tyr-Leu-Ile-Tyr-Ser-Gln-Val-Leu-Phe-Lys-Gly-Gln-Gly-

Cys-Pro-Ser-Thr-His-Val-Leu-Leu-Thr-His-Thr-Ile-Ser-

80
Arg-Ile-Ala-Val-Ser-Tyr-Gln-Thr-Lys-Val-Asn-Leu-Leu-

100
Ser-Ala-Ile-Lys-Ser-Pro-Cys-Gln-Arg-Glu-Thr-Pro-Glu-

Gly-Ala-Glu-Ala-Lys-Pro-Trp-Tyr-Glu-Pro-Ile-Tyr-Leu-

120
Gly-Gly-Val-Phe-Gln-Leu-Glu-Lys-Gly-Asp-Arg-Leu-Ser-

140
Ala-Glu-Ile-Asn-Arg-Pro-Asp-Tyr-Leu-Asp-Phe-Ala-Glu-

153
Ser-Gly-Gln-Val-Tyr-Phe-Gly-Ile-Ile-Ala-Leu.

wherein X represents a hydrogen atom or a methionine residue, together with a suitable pharmaceutical carrier.

31.    Use of the active ingredients of claim 31 for the preparation of a medicament for the treatment of tumors.

## Claims for Austria

1. A method of purifying a polypeptide in which the first 23 NH$_2$-terminal amino acid sequence of the monomeric form is

Ser - Arg - Thr - Pro - Ser - Asp - Lys - Pro - Val -
Ala - His - Val - Val - Ala - Asn - Pro - Gln - Ala -
Glu - Gly - Gln - Leu - Gln

and which has the following characteristics:

(a) a molecular weight of 17,000 ± 1,500 as calculated from data obtained by SDS polyacrylamide electrophoresis;

(b) an isoelectric point of 5.2 ± 0.2 as determined by chromatofocusing;

(c) a cytocidal activity against L-929 cells of $1 \times 10^7$ - $10 \times 10^7$ units/mg protein as defined hereinafter; and

(d) ability to adsorb on a dye adsorption affinity carrier, a molecular weight of 47,000 ± 5,000 as calculated from data obtained by gel permeation chromatography, an isoelectric point of 5.2 - 5.3 as determined by chromatofocusing, and a cytocidal activity against L-929 cells of $1 \times 10^7$ - $10 \times 10^7$ units/mg protein , and which has

antitumor activity or tumor necrotic effect, wherein a dye adsorption affinity carrier is used in the step of purifying said polypeptide from a culture broth containing the same.

2. A method according to Claim 1 wherein any two of the following chromatographic carriers are used in combination: (a) an anion exchanging carrier; (b) a hydrophobic carrier; (c) a chromatofocusing carrier and (d) a gel permeation carrier.

3. A method according to Claim 1 wherein the culture broth containing the polypeptide having antitumor activity or tumor nectrotic effect is obtained by culturing human macrophage-like cells.

4.    A method according to Claim 1  wherein the culture broth containing the polypeptide having antitumor activity or tumor necrotic effect is obtained by culturing a microorganism or cells constructed by the recombinant DNA technology.

5.    A process for preparing a novel polypeptide having antitumor activities which comprises culturing a microorganism transformed by a plasmid comprising a DNA fragment having a promoter region and a ribosome binding (or SD) sequence upstream a DNA sequence coding for the following amino acid sequence so as to express said DNA sequence, extracting a crude polypeptide from the culture, and purifying the crude polypeptide:  said amino acid sequence being Ser-Arg-Thr-Pro-Ser-Asp-Lys-Pro-Val-Ala-His-Val-Val-

20
Ala-Asn-Pro-Gln-Ala-Glu-Gly-Gln-Leu-Gln-Trp-Leu-Asn-

Arg-Arg-Ala-Asn-Ala-Leu-Leu-Ala-Asn-Gly-Val-Glu-Leu-

40
Arg-Asp-Asn-Gln-Leu-Val-Val-Pro-Ser-Glu-Gly-Leu-Tyr-

60
Leu-Ile-Tyr-Ser-Gln-Val-Leu-Phe-Lys-Gly-Gln-Gly-Cys-

Pro-Ser-Thr-His-Val-Leu-Leu-Thr-His-Thr-Ile-Ser-Arg-

80
Ile-Ala-Val-Ser-Tyr-Gln-Thr-Lys-Val-Asn-Leu-Leu-Ser-

100
Ala-Ile-Lys-Ser-Pro-Cys-Gln-Arg-Glu-Thr-Pro-Glu-Gly-

Ala-Glu-Ala-Lys-Pro-Trp-Tyr-Glu-Pro-Ile-Tyr-Leu-Gly-

120
Gly-Val-Phe-Gln-Leu-Glu-Lys-Gly-Asp-Arg-Leu-Ser-Ala-

140
Glu-Ile-Asn-Arg-Pro-Asp-Tyr-Leu-Asp-Phe-Ala-Glu-Ser-

153
Gly-Gln-Val-Tyr-Phe-Gly-Ile-Ile-Ala-Leu.

6.    A process according to Claim 5  wherein the DNA sequence coding for said amino acid sequence is a sequence represented by sequence (I) or a sequence haivng biological functions substantially equivalent thereto:

0205038

```
           10        20        30        40        50
TCTCGAACCCCGAGTGACAAGCCTGTAGCCCATGTTGTAGCAAACCCTCAAG

           60        70        80        90        100
CTGAGGGGCAGCTCCAGTGGCTGAACCGCCGGGCCAATGCCCTCCTGGCCAA

           110       120       130       140       150
TGGCGTGGAGCTGAGAGATAACCAGCTGGTGGTGCCATCAGAGGGCCTGTAC

           160       170       180       190       200
CTCATCTACTCCCAGGTCCTCTTCAAGGGCCAAGGCTGCCCCTCCACCCATG

           210       220       230       240       250       260
TGCTCCTCACCCACACCATCAGCCGCATCGCCGTCTCCTACCAGACCAAGGT

           270       280       290       300       310
CAACCTCCTCTCTGCCATCAAGAGCCCCTGCCAGAGGGAGACCCCAGAGGGG

           320       330       340       350       360
GCTGAGGCCAAGCCCTGGTATGAGCCCATCTATCTGGGAGGGGTCTTCCAGC

           370       380       390       400       410
TGGAGAAGGGTGACCGACTCAGCGCTGAGATCAATCGGCCCGACTATCTCGA

           420      430       440       450       459
CTTTGCCGAGTCTGGGCAGGTCTACTTTGGGATCATTGCCCTG
```

7. A process according to Claim 6 wherein the DNA sequence coding for said amino acid sequence is provided with a triplet coding for translation initiation at the upstream terminal region of sequence (I) and a triplet coding for translation termination at the downstream terminal region thereof.

8. A process according to Claim 7 wherein a transcription terminator coding for termination of transcription which is derived from E. coli is joined just downstream the triplet coding for translation terminaiton.

9. A process according to Claim 8 wherein the transcription terminator is a TrpA terminator.

10. A process according to Claim 8 or 9 wherein the DNA sequence which has the transcription terminator derived from E. coli and is joined just downstream the triplet coding for termination of translation is represented by the following sequence:

0205038

ATGTCTCGAACCCCGAGTGACAAGCCTGTAGCCCATG

TTGTAGCAAACCCTCAAGCTGAGGGGCAGCTCCAGTG

GCTGAACCGCCGGGCCAATGCCCTCCTGGCCAATGGC

GTGGAGCTGAGAGATAACCAGCTGGTGGTGCCATCAG

AGGGCCTGTACCTCATCTACTCCCAGGTCCTCTTCAA

GGGCCAAGGCTGCCCCTCCACCCATGTGCTCCTCACC

CACACCATCAGCCGCATCGCCGTCTCCTACCAGACCA

AGGTCAACCTCCTCTCTGCCATCAAGAGCCCCTGCCA

GAGGGAGACCCCAGAGGGGGCTGAGGCCAAGCCCTGG

TATGAGCCCATCTATCTGGGAGGGGTCTTCCAGCTGG

AGAAGGGTGACCGACTCAGCGCTGAGATCAATCGGCC

CGACTATCTCGACTTTGCCGAGTCTGGGCAGGTCTAC

TTTGGGATCATTGCCCTGTGAG⌐TCGACAGCCCGCCT

$\overset{\downarrow}{S}$

AATGAGCGGGCTTTTTTTTTCTCGG⌐AATTCTCATGT

$\overset{\downarrow}{E}$

TTGACAGCTTATCATCGATAAGCTTTAATGCGGTAG

wherein ATG represents the triplet coding for translation initiation, TGA represents the triplet coding for translation termination, ↔ represents a TrpA terminatror, $\overset{\downarrow}{S}$ represents the cutting site with SalI and $\overset{\downarrow}{E}$ represents the cutting site with EcoRI.

11. A process according to Claim 5 wherein the promoter region comprises a $P_L$ promoter originating from λ phage or a $T_4$ phage promoter or both of them.

12. A process according to Claim 5 wherein the promoter region is derived from a gene coding for klipoprotein of the outer cell membrane of E. coli.

13. A process according to Claim 5 wherein the ribosome binding (or SD) sequence is a DNA sequence derived from $T_4$ phage or a chemically synthesized DNA sequence.

14. A process according to Claim 5 wherein the plasmid is E. coli plasmid pBR322 from which the rop (repressor of primer) DNA region controlling replication of the plastic DNA is removed.

15. A process according to Claim 5 wherein the plasmid is $pPLT4^{\Delta N4}TNFST8$, $pT4^{\Delta N4}TNFST8^{rop-}$ or $pIN5^{\Delta N4}TNFST8^{rop-}$.

16.    A process according to Claim 5  wherein the microorganism is E. coli.

17.    Escherichia coli transformed by a plasmid represented by $pPLT4^{\Delta N4}TNFST8$, $pT4^{\Delta N4}TNFST8^{rop-}$ or $pIN5^{\Delta N4}TNFST8^{rop-}$.

18.    Escherichia coli according to Claim 17 which is represented by $WA802/CI/pPLT4^{\Delta N4}TNFST8$, $W3110/CI/pPLT4^{\Delta N4}TNFST8$, $WA802/pT4^{\Delta N4}TNFST8^{rop-}$, $W3110/pT4^{\Delta N4}TNFST8^{rop-}$, $WA802/pIN5^{\Delta N4}TNFST8^{rop-}$ or $W3110/pIN5^{\Delta N4}TNFST8^{rop-}$.

19.    Use of a polypeptide having the follow-ing amino acid sequence:

        X-Ser-Arg-Thr-Pro-Ser-Asp-Lys-Pro-Val-Ala-His-Val-

                                    20
        Val-Ala-Asn-Pro-Gln-Ala-Glu-Gly-Gln-Leu-Gln-Trp-Leu-

        Asn-Arg-Arg-Ala-Asn-Ala-Leu-Leu-Ala-Asn-Gly-Val-Glu-

            40
        Leu-Arg-Asp-Asn-Gln-Leu-Val-Val-Pro-Ser-Glu-Gly-Leu-

                                        60
        Tyr-Leu-Ile-Tyr-Ser-Gln-Val-Leu-Phe-Lys-Gly-Gln-Gly-

        Cys-Pro-Ser-Thr-His-Val-Leu-Leu-Thr-His-Thr-Ile-Ser-

                80
        Arg-Ile-Ala-Val-Ser-Tyr-Gln-Thr-Lys-Val-Asn-Leu-Leu-

                                        100
        Ser-Ala-Ile-Lys-Ser-Pro-Cys-Gln-Arg-Glu-Thr-Pro-Glu-

        Gly-Ala-Glu-Ala-Lys-Pro-Trp-Tyr-Glu-Pro-Ile-Tyr-Leu-

            120
        Gly-Gly-Val-Phe-Gln-Leu-Glu-Lys-Gly-Asp-Arg-Leu-Ser-

                                        140
        Ala-Glu-Ile-Asn-Arg-Pro-Asp-Tyr-Leu-Asp-Phe-Ala-Glu-

                                    153
        Ser-Gly-Gln-Val-Tyr-Phe-Gly-Ile-Ile-Ala-Leu.

wherein X represetns a hydrogen atom or a methionine residue  in the preparation of a medicament for the treatment of tumors.

## Fig. 1

met-ser-thr-glu-ser-met-ile-arg-asp-val-glu-leu-ala-glu-glu-ala-leu-pro-lys-lys- 20

thr-gly-gly-pro-gln-gly-ser-arg-arg-cys-leu-phe-leu-ser-leu-phe-ser-phe-leu-ile- 40

val-ala-gly-ala-thr-thr-leu-phe-cys-leu-leu-his-phe-gly-val-ile-gly-pro-gln-arg- 60

glu-glu-phe-pro-arg-asp-leu-ser-leu-ile-ser-pro-leu-ala-gln-ala-Val-Arg-Ser-Ser- 80

Ser-Arg-Thr-Pro-Ser-Asp-Lys-Pro-Val-Ala-His-Val-Val-Ala-Asn-Pro-Gln-Ala-Glu-Gly- 100

Gln-Leu-Gln-Trp-Leu-Asn-Arg-Arg-Ala-Asn-Ala-Leu-Leu-Ala-Asn-Gly-Val-Glu-Leu-Arg- 120

Asp-Asn-Gln-Leu-Val-Val-Pro-Ser-Glu-Gly-Leu-Tyr-Leu-Ile-Tyr-Ser-Gln-Val-Leu-Phe- 140

Lys-Gly-Gln-Gly-Cys-Pro-Ser-Thr-His-Val-Leu-Leu-Thr-His-Thr-Ile-Ser-Arg-Ile-Ala- 160

Val-Ser-Tyr-Gln-Thr-Lys-Val-Asn-Leu-Leu-Ser-Ala-Ile-Lys-Ser-Pro-Cys-Gln-Arg-Glu- 180

Thr-Pro-Glu-Gly-Ala-Glu-Ala-Lys-Pro-Trp-Tyr-Glu-Pro-Ile-Tyr-Leu-Gly-Gly-Val-Phe- 200

Gln-Leu-Glu-Lys-Gly-Asp-Arg-Leu-Ser-Ala-Glu-Ile-Asn-Arg-Pro-Asp-Tyr-Leu-Asp-Phe- 220

Ala-Glu-Ser-Gly-Gln-Val-Tyr-Phe-Gly-Ile-Ile-Ala-Leu 233

Fig. 2

COLUMN CHROMATOGRAPHY OF U-937 TNF
ON DEAE-SEPHAROSE

FRUCTION NO.

Fig. 3

CHROMATOFOCUSING OF U-937 TNF

Fig. 4

COLUMN CHROMATOGRAPHY OF U-937 TNF
ON PHENYL-SEPHAROSE

# Fig. 5

COLUMN CHROMATOGRAPHY OF U-937 TNF
ON MATREX BLUE A

# Fig. 6

GEL PERMIATION CHROMATOGRAPHY OF TNF

SDS-PAGE PATTERNS OF
U-937 TNF AND RECOMBINANT
TNF

*Fig. 7*

← 94.0K (PHOSPHORYLASE b)

← 67.0K (BOVINE SERUM ALBUMINE)

← 43.0K (OVALBUMIN)

← 30.0K (CARBONATE DEHYDRATASE)

← 20.1K (SOYBEAN TRYPSIN
    INHIBITOR)

← 14.4 (α-LACTALBUMIN)

STANDARD

RECOMBINANT TNF

U-937 TNF +RECOMBINANT

U-937 TNF

RECOMBINANT TNF

STANDARD

# Fig. 8

# Fig. 9 a

*Fig. 9b*

# Fig. 10

Fig. 11

13/26

0205038

Fig.12

COLUMN CHROMATOGRAPHY ON
DEAE- TOYOPEAL 650C

# Fig. 13

COLUMN CHROMATOGRAPHY ON
DEAE- SEPHAROSE CL-6B

0205038

# Fig. 14

COLUMN CHROMATOGRAPHY ON
MATREX BLUE A

Fig. 15a

HEAT STABILITY OF TNF (U-937)

# Fig. 15b

HEAT STABILITY

0205038

# Fig. 16 a

pH STABILITY OF TNF (U-937)

# Fig. 16 b

## pH STABILITY

## Fig. 17

COLUMN CHROMATOGRAPHY OF RECOMBINANT
TNF ON DEAE-SEPHAROSE

# Fig. 18

COLUMN CHROMATOGRAPHY OF RECOMBINANT TNF
ON MATREX·BLUE A

Fig. 19

COLUMN CHROMATOGRAPHY OF RECOMBINANT TNF ON PHENYL-SEPHAROSE CL-AB

## Fig. 20

COLUMN CHROMATOGRAPHY OF RECOMBINANT
DNA OR SEPHACRYL S-200

# Fig. 2I

SDS PAGE PATERNS
OF RECOMBINANT TNF

← 94.0 (PHOSPHORYLASE b )
← 62.0 ( BOVINE SERUM ALBUMIN )
← 43.0 ( OVALBMIN )

← 30.0 (CARBONATE DEHYDRATASE )

← 20.1K (SOYBEAN TRYPSIN INHIBITOR )

← 14 4K (α-LACTALBUMIN )

↑ STANDARD
↑ RECOMBINANT TNF (TNF)
↑ RECOMBINANT TNF(ΔTNF)
↑ RECOMBINANT TNF (TNF+ΔTNF)
↑ STANDARD

# Fig. 22 a

CHROMATOFOCUSING OF TNF (157 AMINO ACIDS)

# Fig. 22 b

CHROMATOFOCUSING OF ΔTNF

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | NATURE, vol. 313, no. 6005, February/March 1985, pages 803-806, London, GB; T. SHIRAI et al.: "Cloning and expression in Escherichia coli of the gene for human tumour necrosis factor" * Whole document * | 1 | C 12 P 21/02 C 12 N 15/00 A 61 K 37/02 C 12 N 1/20 (C 12 N 1/20 C 12 R 1:19 ) |
| D,A | SCIENCE, vol. 228, 12th April 1985, pages 149-154; A.M. WANG et al.: "Molecular cloning of the complementary DNA for human tumor necrosis factor" * Whole document * | 1 | |
| D,A | NATURE, vol. 312, no. 5996, December 1984/January 1985, pages 724-729, London, GB; D. PENNICA et al.: "Human tumour necrosis factor: precursor structure, expression and homology to lymphotoxin" * Whole document * | 1 | TECHNICAL FIELDS SEARCHED (Int. Cl.4) C 12 P C 12 N |
| A | EP-A-0 131 789 (SLOAN-KETTERING INSTITUTE FOR CANCER RESEARCH) * Claims * | 1 | |
| A | EP-A-0 100 641 (GENENTECH) * Claims * | 1 | |

The present search report has been drawn up for all claims

| Place of search | Date of completion of the search | Examiner |
|---|---|---|
| THE HAGUE | 05-09-1986 | DELANGHE L.L.M. |